# EUROPEAN PATENT APPLICATION

(11) **EP 4 144 765 A1**
(43) Date of publication of application: **08.03.2023**
(21) Application number: 21797718.0
(22) Date of filing: 30.04.2021
(51) Int. Cl.: C07K 19/00, C12N 15/62, G01N 33/58

(54) **DETECTION REAGENT FOR SCREENING BLOCKING AGENT OF CORONAVIRUS INFECTIONS, AND DETECTION METHOD**

(30) Priority: 30.04.2020 CN 202010365755
(71) Applicant: Yang Sheng Tang Company, Ltd., Hangzhou, Zhejiang 310000 (CN); Xiamen University, Xiamen, Fujian 361005 (CN)
(72) Inventor: ZHANG, Yali, Xiamen Fujian 361005 (CN); WANG, Shaojuan, Xiamen Fujian 361005 (CN); WU, Yangtao, Xiamen Fujian 361005 (CN); HOU, Wangheng, Xiamen Fujian 361005 (CN); YE, Jianghui, Xiamen Fujian 361005 (CN); WANG, Juan, Xiamen Fujian 361005 (CN); ZHANG, Tianying, Xiamen Fujian 361005 (CN); CHENG, Tong, Xiamen Fujian 361005 (CN); YUAN, Quan, Xiamen Fujian 361005 (CN); XIA, Ningshao, Xiamen Fujian 361005 (CN)
(74) Representative: Held, Stephan
(86) International application number: PCT/CN2021/091347
(87) International publication number: WO 2021/219121

(57) **Abstract**

Disclosed are a fusion protein probe and a cell model for screening a blocking agent of coronavirus infections, a screening system comprising same, and a method of using the screening system for screening a blocking agent of coronavirus infections. The screening system and method do not involve live viruses, are simple and convenient to operate, have a high accuracy, are suitable for high throughput screening, and are of great significance for the development of coronavirus neutralizing antibodies, preventive vaccines, and small molecule drugs.

## Description

### Technical Field

The present invention relates to the field of virology. Specifically, the present invention provides a fusion protein probe, a cell model, and a screening system comprising the same for screening a blocking agent of coronavirus infection. The present invention also provides a detection method for screening blocking agent of coronavirus infection using the screening system.

### Background Art

Coronavirus infection can cause respiratory diseases in humans, mild coronavirus infection can cause flu-like symptoms, and severe infection can develop into severe viral pneumonia, threatening human life and health. Coronavirus can infect humans and animals at the same time. If some animal-derived coronaviruses break through the host barrier and infect humans, they may spread rapidly in the population and cause serious diseases.

Severe acute respiratory syndrome (SARS-CoV-1), Middle East respiratory syndrome (MERS), and coronavirus disease 2019 (COVID-2019) caused by severe acute respiratory syndrome coronavirus 2 (SARS-CoV-2) infection all are typical cases of animal-derived coronaviruses infecting humans with serious consequences. The development of drugs such as antibodies, vaccines (which stimulate the body to produce antibodies after immunization) and small molecule compounds that can block coronavirus infection in humans is crucial for the prevention and control of coronavirus infection and related diseases in humans.

In the process of virus infection of host cells, coronaviruses, including SARS-CoV-2, cause the infection through the fusion of the viral membrane and the cellular membrane mediated by the binding of the spike protein (spike) on the surface of the membrane of the virus to the cell receptor, endocytosis, and releasing the viral genome into cells. The spike protein of coronavirus is a class I fusion protein that functions as a trimer composed of three monomeric protein molecules. The protein contains two subunits, named S1 and S2 respectively. The S1 subunit contains the receptor-binding domain (receptor binding site, RBD), which is responsible for interacting with cell receptors; while the S2 subunit mainly contains the basic functional elements that mediate virus/cell membrane fusion, such as one fusion peptide, two heptad repeats (HRs), one membrane proximal external region (MPER) and a transmembrane domain (TMD). The RBD in the S1 protein is the domain by which the virus directly and specifically binds to the cell receptor, and is the most critical for the virus to infect cells. According to research, the cell receptors of coronaviruses identified so far mainly include aminopeptidase N (APN), angiotensin converting enzyme II (ACE2), dipeptidyl peptidase 4 (DPP4), 9-O-acetylated sialic acid (9-O-Ac-Sia), etc., of which ACE2 is the receptor of SARS-CoV-1 and SARS-CoV-2, and DPP4 is the receptor of MERS, and 9-O-Ac-Sia is considered to be the receptor for the coronavirus HCoV-HKU1.

The development of therapeutic antibodies, vaccines and therapeutic drugs specific for SARS-CoV-2 is an important research direction for the prevention and control of the SARS-CoV-2 epidemic. For respiratory viruses such as SARS-CoV-1 and SARS-CoV-2 that infect humans and can cause severe diseases, the use of authentic virus infection models to evaluate the function of infection blocking agents such as antibodies requires biosafety level 3 or higher laboratories, so that the cost of research is extremely high, and it is difficult to conduct high-throughput and large-scale functional screening, which greatly restricts the process of vaccine and drug development. The use of pseudotype virus with surface membrane of coronavirus is an alternative solution often used in previous studies, but it still needs to be operated in a biosafety level 2 or above laboratory. In addition, because virus infection and replication require a certain time, it often takes more than 24-72 hours of culture from the infection to the actual detection of infection indicators, which requires a long time to occupy cell culture devices and other equipment, thereby reducing efficiency. Therefore, it is of great significance to develop a detection method and reagent, which is easy to operate, has high accuracy, does not involve live viruses, and is suitable for high-throughput screening, for the development of neutralizing antibodies, preventive vaccines, and small-molecule drugs for coronaviruses.

### Contents of the present invention

In order to overcome the technical limitations of the existing screening systems for antiviral activity, the present invention successfully established a novel screening system that can evaluate the effect of an inhibitor for blocking coronavirus infection under virus-free conditions, which is of great significance for diagnosis, prevention and treatment of coronavirus (e.g., SARS-CoV-2).

### Fusion protein and multimer

In a first aspect, the present invention provides a fusion protein, which comprises a S protein receptor-binding domain (RBD) of a coronavirus and a fluorescent protein.

In certain embodiments, the coronavirus is selected from SARS-CoV-2, SARS-CoV-1, MERS-CoV, HKU1-CoV, or RaTG13.

In certain embodiments, the fusion protein comprises the S protein receptor-binding domain and the fluorescent protein from the N-terminal to the C-terminal.

In certain embodiments, the S protein receptor-binding domain (RBD) of SARS-CoV-2 comprises: (i) an sequence set forth in SEQ ID NO:1, or (ii) a sequence having a sequence identity of at least 80%, at least 85%, at least 90%, at least 91%, at least 92%, at least 93%, at least 94%, at least 95%, at least 96%, at least 97%, at least 98%, or at least 99% as compared to the sequence set forth in SEQ ID NO:1, or (iii) a sequence having a substitution, deletion or addition of one or several amino acids (e.g., a substitution, deletion or addition of 1, 2, 3, 4 or 5 amino acids) as compared to the sequence set forth in SEQ ID NO: 1. In certain embodiments, the S protein receptor-binding domain (RBD) of SARS-CoV-2 is encoded by the sequence set forth in SEQ ID NO:2.

In certain embodiments, the S protein receptor-binding domain (RBD) of SARS-CoV-1 comprises: (i) a sequence consisting of amino acid residues at positions 21 to 256 of SEQ ID NO: 13, or (ii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to (i), or (iii) a sequence having a substitution, deletion or addition of one or several amino acids (e.g., a substitution, deletion or addition of 1, 2, 3, 4 or 5 amino acids) as compared to (i).

In certain embodiments, the S protein receptor-binding domain (RBD) of MERS-CoV comprises: (i) a sequence consisting of amino acid residues at positions 21 to 274 of SEQ ID NO: 15, or (ii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence set forth in (i), or (iii) a sequence having a substitution, deletion or addition of one or several amino acids (e.g., a substitution, deletion or addition of 1, 2, 3, 4 or 5 amino acids) as compared to the sequence set forth in (i).

In certain embodiments, the S protein receptor-binding domain (RBD) of HKU1-CoV comprises: (i) a sequence consisting of amino acid residues at positions 21 to 349 of SEQ ID NO: 17, or (ii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence set forth in (i), or (iii) a sequence having a substitution, deletion or addition of one or several amino acids (e.g., a substitution, deletion or addition of 1, 2, 3, 4 or 5 amino acids) as compared to the sequence set forth in (i).

In certain embodiments, the S protein receptor-binding domain (RBD) of RaTG13 comprises: (i) a sequence consisting of amino acid residues at positions 21 to 257 of SEQ ID NO: 19, or (ii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence set forth in (i), or (iii) a sequence having a substitution, deletion or addition of one or several amino acids (e.g., a substitution, deletion or addition of 1, 2, 3, 4 or 5 amino acids) as compared to the sequence set forth in (i).

In certain embodiments, the S protein receptor-binding domain and the fluorescent protein are optionally linked by a peptide linker. In certain embodiments, the peptide linker is a flexible peptide linker, for example, comprising 1 to 15 (e.g., 1 to 12, 1 to 10) contiguous amino acid residues that are identical or different and selected from glycine and serine. In certain embodiments, the peptide linker is (GₘS)ₙ, wherein m is an integer selected from 1 to 4 and n is an integer selected from 1 to 3. In certain exemplary embodiments, the peptide linker comprises a sequence set forth in SEQ ID NO:6.

In certain embodiments, the fluorescent protein is selected from the group consisting of green fluorescent protein, blue fluorescent protein, cyan fluorescent protein, yellow fluorescent protein, orange or red fluorescent protein, near-infrared fluorescent protein, or long Stokes shift fluorescent protein.

In certain embodiments, the fluorescent protein is selected from the group consisting of green fluorescent proteins, such as mGamillus, mNeonGreen, EGFP, mClover, UnaG, TurboGFP, TagGFP, Venus, EYFP, RFP, iRFP670, mBeRFP, CyOFP1.

In certain embodiments, the fluorescent protein comprises a sequence set forth in SEQ ID NO: 38 or 39.

In certain exemplary embodiments, the fusion protein comprises an amino acid sequence selected from the group consisting of:
(1) an amino acid sequence consisting of the amino acid residues at positions 21 to 516 of the sequence set forth in SEQ ID NO: 7;
(2) an amino acid sequence consisting of the amino acid residues at positions 21 to 514 of the sequence set forth in SEQ ID NO: 8;
(3) an amino acid sequence consisting of the amino acid residues at positions 21 to 515 of the sequence set forth in SEQ ID NO: 13;
(4) an amino acid sequence consisting of the amino acid residues at positions 21 to 533 of the sequence set forth in SEQ ID NO: 15;
(5) an amino acid sequence consisting of the amino acid residues at positions 21 to 608 of the sequence set forth in SEQ ID NO: 17; or
(6) An amino acid sequence consisting of the amino acid residues of positions 21 to 516 of the sequence set forth in SEQ ID NO: 19.

In certain embodiments, the fusion protein further comprises a signal peptide and/or a tag protein.

In certain embodiments, the fusion protein comprises a signal peptide at its N-terminal.

In certain embodiments, the signal peptide is a B2M signal peptide, for example, a signal peptide set forth in SEQ ID NO:37.

In certain embodiments, the fusion protein comprises a tag protein, such as a His tag, at its C-terminal.

In certain exemplary embodiments, the fusion protein comprises an amino acid sequence set forth in any one of SEQ ID NOs: 7, 8, 13, 15, 17, 19. In certain exemplary embodiments, the fusion protein is encoded by a sequence set forth in any one of SEQ ID NOs: 9, 10, 14, 16, 18, 20.

In a second aspect, the present invention provides a fusion protein, which comprises a S protein ectodomain sequence of a coronavirus, a trimerization domain sequence, and a fluorescent protein.

In certain embodiments, the fusion protein comprises, the S protein ectodomain sequence, the trimerization domain sequence, and the fluorescent protein, from the N-terminal to the C-terminal.

In certain embodiments, the coronavirus is SARS-CoV-2.

In certain embodiments, the S protein ectodomain sequence is selected from the amino acid sequences shown below:
(i) a sequence set forth in SEQ ID NO: 21;
(ii) a sequence having a substitution, deletion or addition of one or several amino acids (e.g., a substitution, deletion or addition of 1, 2, 3, 4, 5, 6, 7, 8, 9 or 10 amino acids) as compared to the sequence set forth in SEQ ID NO: 21; or
(iii) a sequence having a sequence identity of at least 80%, at least 85%, at least 90%, at least 91%, at least 92%, at least 93%, at least 94%, at least 95%, at least 96%, at least 97%, at least 98%, or at least 99% as compared to the sequence set forth in SEQ ID NO: 21.

In certain embodiments, the S protein ectodomain sequence is encoded by a sequence set forth in SEQ ID NO:22.

In certain embodiments, the trimerization domain sequence comprises a sequence set forth in SEQ ID NO:40. In certain embodiments, the trimerization domain sequence is encoded by a sequence set forth in SEQ ID NO:23.

In certain embodiments, the trimerization domain sequence and the fluorescent protein are optionally linked by a peptide linker (e.g., a flexible peptide linker). In certain embodiments, the peptide linker comprises 1 to 15 (e.g., 1 to 12, such as 1 to 10) contiguous amino acid residues that are identical or different and selected from glycine and serine. In certain embodiments, the peptide linker is (GₘS)ₙ, wherein m is an integer selected from 1 to 4 and n is an integer selected from 1 to 3; preferably, the peptide linker comprises a sequence set forth in SEQ ID NO: 6.

In certain embodiments, the fluorescent protein is selected from the group consisting of green fluorescent protein, blue fluorescent protein, cyan fluorescent protein, yellow fluorescent protein, orange or red fluorescent protein, near-infrared fluorescent protein, or long Stokes shift fluorescent protein.

In certain embodiments, the fluorescent protein is selected from the group consisting of green fluorescent proteins, such as mGamillus, mNeonGreen, EGFP, mClover, UnaG, TurboGFP, TagGFP, Venus, EYFP, RFP, iRFP670, mBeRFP, CyOFP1. In certain embodiments, the fluorescent protein comprises a sequence set forth in SEQ ID NO: 38 or 39.

In certain exemplary embodiments, the fusion protein comprises an amino acid sequence selected from the group consisting of:
(1) an amino acid sequence consisting of amino acid residues at positions 21 to 1502 of the sequence set forth in SEQ ID NO: 26; or
(2) an amino acid sequence consisting of amino acid residues at positions 21 to 1499 of the sequence set forth in SEQ ID NO: 27.

In certain embodiments, the fusion protein further comprises a signal peptide and/or a tag protein.

In certain embodiments, the fusion protein comprises a signal peptide at its N-terminal.

In certain embodiments, the signal peptide is a B2M signal peptide, such as a signal peptide set forth in SEQ ID NO:37.

In certain embodiments, the fusion protein comprises a tag protein, such as a His tag, at its C-terminal.

In certain exemplary embodiments, the fusion protein comprises an amino acid sequence set forth in SEQ ID NO: 26 or 27. In certain exemplary embodiments, the fusion protein is encoded by a sequence set forth in SEQ ID NO: 24 or 25.

In a third aspect, the present invention provides a multimer, which comprises the fusion protein of the second aspect. In certain embodiments, the multimer is a homomultimer. In certain embodiments, the multimer is a trimer. In certain embodiments, the multimer is a trimer formed from the same fusion protein.

### Preparation of fusion protein and multimer

The fusion protein of the first aspect or the second aspect of the present invention can be prepared by various methods known in the art, for example, produced by genetic engineering method (recombinant technology), or by chemical synthesis method (e.g., Fmoc solid-phase method). The fusion protein of the present invention is not limited by the manner in which it is produced.

Accordingly, in another aspect, the present invention provides an isolated nucleic acid molecule, which comprises a nucleotide sequence encoding the fusion protein of the first aspect or the second aspect.

In another aspect, the present invention provides a vector (e.g., a cloning vector or an expression vector), which comprises the isolated nucleic acid molecule as described above. In certain embodiments, the vector is, for example, a plasmid, cosmid, phage, and the like.

In another aspect, the present invention provides a host cell, which comprises the isolated nucleic acid molecule or the vector as described above. Such host cell includes, but is not limited to, prokaryotic cell such as E. coli cell, and eukaryotic cell such as yeast cell, insect cell, plant cell, and animal cell (e.g., mammalian cell, such as mouse cell, human cell, etc.).

In another aspect, there is provided a method for preparing the fusion protein of the first aspect of the present invention, comprising, culturing a host cell comprising a nucleotide sequence encoding the fusion protein of the first aspect under conditions that allow the expression of the protein, and recovering the fusion protein from a cultured host cell culture.

In another aspect, there is provided a method for preparing the fusion protein of the second aspect of the present invention or the multimer of the third aspect, comprising, culturing a host cell comprising a nucleotide sequence encoding the fusion protein of the second aspect under conditions that allow the expression of the protein, and recovering the multimer from a cultured host cell culture, wherein the fusion protein exists in the form of a multimer.

### Kit

In a fourth aspect, the present invention provides a kit, which comprises: the fusion protein of the first aspect; or, (ii) the fusion protein of the second aspect or the multimer of the third aspect.

In certain embodiments, the kit further comprises a cell expressing a recombinant coronavirus receptor, the recombinant coronavirus receptor comprises a coronavirus receptor and a fluorescent protein fused thereto. In this article, the expression "coronavirus receptor" refers to a cell receptor of coronavirus, and the virus enters the host cell by binding to the corresponding cell receptor on the surface of the host cell, resulting in a membrane fusion reaction.

In certain embodiments, the coronavirus receptor is selected from ACE2, DPP4, APN, and the like.

In certain embodiments, the coronavirus is selected from SARS-CoV-2 and/or SARS-CoV-1, and the coronavirus receptor is ACE2.

In certain embodiments, the coronavirus is selected from MERS-CoV and the recombinant coronavirus receptor is DPP4.

In certain embodiments, the recombinant coronavirus receptor comprises the coronavirus receptor and the fluorescent protein from the N-terminal to the C-terminal.

In certain embodiments, the cell stably expresses the recombinant coronavirus receptor.

In certain embodiments, the cell expresses the recombinant coronavirus receptor on its surface.

In certain embodiments, the cell expressing the recombinant coronavirus receptor is prepared by introduction (e.g., lentiviral transfection or transposon transfection) of a nucleotide sequence encoding the recombinant coronavirus receptor into a host cell. In certain embodiments, the host cell does not natively express the coronavirus receptor.

In certain embodiments, the cell comprises a nucleotide sequence encoding the recombinant coronavirus receptor.

In certain embodiments, the cell is an adherent cell, such as 293T or H1299 cell.

In certain embodiments, the coronavirus receptor is an ACE2 protein, such as a human ACE2 protein.

In certain embodiments, the human ACE2 protein comprises a sequence set forth in SEQ ID NO:41. In certain embodiments, the human ACE2 protein is encoded by a sequence set forth in SEQ ID NO:30.

In certain embodiments, the fluorescent protein is selected from the group consisting of green fluorescent protein, blue fluorescent protein, cyan fluorescent protein, yellow fluorescent protein, orange or red fluorescent protein, near-infrared fluorescent protein, or long Stokes shift fluorescent protein.

In certain embodiments, the fluorescent protein is selected from red fluorescent protein, near-infrared fluorescent protein, or long Stokes shift fluorescent protein, such as mRuby3, mApple, FusionRed, mCherry, mScarlet, RFP, iRFP670, mBeRFP, or CyOFP1.

In certain embodiments, the fluorescent protein comprises a sequence set forth in SEQ ID NO:42.

In certain embodiments, the recombinant coronavirus receptor comprises a sequence set forth in SEQ ID NO:32.

In certain embodiments, the recombinant coronavirus receptor is encoded by a sequence set forth in SEQ ID NO:31.

In certain embodiments, the cell comprises a sequence set forth in SEQ ID NO:31.

In certain embodiments, the kit of the present invention comprises: the fusion protein described in the first aspect, and the cell expressing the recombinant coronavirus receptor described above. In certain embodiments, the fluorescent protein contained in the fusion protein is detectably different from the fluorescent protein in the recombinant coronavirus receptor expressed by the cell. In certain embodiments, the fluorescent protein contained in the fusion protein is green fluorescent protein. In certain embodiments, the fluorescent protein in the recombinant coronavirus receptor expressed by the cell is selected from red fluorescent protein, near-infrared fluorescent protein, or long Stokes shift fluorescent protein.

In certain embodiments, the kit of the present invention comprises: the fusion protein described in the second aspect or the multimer (e.g., trimer) described in the third aspect, and the cell expressing the recombinant coronavirus receptor described above. In certain embodiments, the fluorescent protein contained in the monomer forming the multimer is detectably different from the fluorescent protein in the recombinant coronavirus receptor expressed by the cell. In certain embodiments, the fluorescent protein contained in the monomer forming the multimer is green fluorescent protein. In certain embodiments, the fluorescent protein in the recombinant coronavirus receptor expressed by the cell is selected from red fluorescent protein, near-infrared fluorescent protein, or long Stokes shift fluorescent protein.

In certain embodiments, the kit of the present invention further comprises a solid support. In certain embodiments, the solid support is selected from microtiter plates (e.g., microwell plate or ELISA plate). In certain embodiments, the solid support is suitable for fluorescence measurement. In certain embodiments, the cell expressing the recombinant coronavirus receptor is immobilized on the surface of the solid support.

### Detection use and method

In a fifth aspect, the present invention provides a method for evaluating a fusion inhibitory activity of a fusion inhibitor against a coronavirus, and/or for screening a fusion inhibitor against a coronavirus, which comprises using the fusion protein of the first aspect, the fusion protein of the second aspect or the multimer of the third aspect, or the kit of the fourth aspect.

In certain embodiments, the method comprises:
(1) in the presence of a reagent to be tested, contacting a detection reagent with a cell expressing the recombinant coronavirus receptor defined in the fourth aspect of the present invention, in which the detection reagent is selected from: the fusion protein of the first aspect or the multimer of the third aspect; wherein, the fluorescent protein contained in the detection reagent is detectably different from the fluorescent protein in the recombinant coronavirus receptor expressed by the cell; and, the coronavirus receptor contained in the recombinant coronavirus receptor is a cell receptor of coronavirus to which the fusion inhibitor is directed;
(2) measuring a fluorescence intensity of a cytoplasmic region of the cell, wherein the fluorescence intensity is a fluorescence intensity of the fluorescent protein contained in the detection reagent.

In certain embodiments, the method further comprises: generating a dose-response curve of the reagent to be tested based on the fluorescence intensity value obtained in step (2) and obtaining an EC₅₀ therefrom; evaluating the fusion inhibitory activity of the reagent to be tested against the coronavirus according to the EC₅₀. In certain embodiments, the EC₅₀ is obtained by the following method: repeating steps (1) to (2) with a series of samples comprising varying amounts of the reagent to be tested, thereby generating a dose-response curve for the reagent to be tested and thereby determining EC₅₀.

In certain embodiments, the method further comprises:
(3) comparing the measured value in step (2) with the fluorescence intensity measured in the absence of the reagent to be tested, and obtaining the following ratio: (measured value in the absence of the reagent to be tested - measured value in step (2)) / measured value in the absence of the reagent to be tested.

In certain embodiments, the method further comprises:
(4) generating a dose-response curve of the reagent to be tested based on the ratio obtained in step (3) and thereby obtaining EC₅₀; evaluating the fusion inhibitory activity of the reagent to be tested against the coronavirus according to the EC₅₀. In certain embodiments, the EC₅₀ of step (4) is obtained by the following method: repeating steps (1) to (3) with a series of samples comprising varying amounts of the reagent to be tested, thereby generating a dose-response curve of the reagent to be tested and determining EC₅₀ therefrom.

In certain embodiments, the cell described in step (1) is immobilized on the surface of a solid support. In certain embodiments, the solid support is selected from microtiter plates (e.g., microwell plate or ELISA plate). In certain embodiments, the solid support is suitable for fluorescence measurement (e.g., microwell plate with black wall and transparent bottom).

In certain embodiments, the number of cells described in step (1) and step (2) is multiple. In certain embodiments, the number of cells determined in step (2) is not less than 100, such as not less than 500, not less than 800, or not less than 1000. In certain embodiments, the fluorescence intensity measured in step (2) is the mean fluorescence intensity.

In certain embodiments, step (2) may further comprise: determining the total fluorescence intensity of the cell, the fluorescence intensity is the fluorescence intensity of the fluorescent protein contained in the fusion protein or multimer; and, comparing the fluorescence intensity of the cytoplasmic region with the total fluorescence intensity of the cell to which it belongs, and obtaining the ratio of the two, in which the ratio can be used as the ratio reflecting cell uptake of probe, and used as the measured value obtained in step (2) for subsequent analysis.

In certain embodiments, step (2) further comprises: measuring the fluorescence intensity of the cell membrane region of the cell, wherein the fluorescence intensity is the fluorescence intensity of the fluorescent protein contained in the recombinant coronavirus receptor expressed by the cell. In certain embodiments, the fluorescence intensity is the mean fluorescence intensity.

In certain embodiments, step (2) further comprises comparing the fluorescence intensity of the cell membrane region between different batches of experiments, or between different test wells in the same batch, wherein the fluorescence intensity is the fluorescence intensity of the fluorescent protein contained in the recombinant coronavirus receptor expressed by the cell, and the values can be used for correction of variation between different batches or different test wells.

In certain embodiments, step (2) further comprises comparing the fluorescence intensity of the cytoplasmic region with the fluorescence intensity of the cell membrane region, and obtaining a ratio between the two, which is used as a calibrated measured value for subsequent step; wherein, the fluorescence intensity of the cytoplasmic region is the fluorescence intensity of the fluorescent protein contained in the fusion protein or multimer, and the fluorescence intensity of the cell membrane region is the fluorescence intensity of the fluorescent protein contained in the recombinant coronavirus receptor. In such embodiments, the calibrated value can be used for calibration of batch-to-batch assays or for calibration between different wells in the same batch to reduce or eliminate the effects of possible variation of batch-to-batch or well-to-well.

In certain embodiments, step (2) is determined by a fluorescence microscopy or a high content imaging system.

In certain embodiments, no washing step is comprised between steps (1) and (2).

In certain embodiments, the coronavirus receptor is selected from the group consisting of ACE2, DPP4, APN, and the like.

In certain embodiments, the coronavirus receptor is ACE2 (e.g., human ACE2). In certain embodiments, the coronavirus uses ACE2 (e.g., human ACE2) as a cell receptor. In certain embodiments, the coronavirus is selected from SARS-CoV-2 and/or SARS-CoV-1. In certain embodiments, the coronavirus is SARS-CoV-2.

In certain embodiments, the fusion inhibitor against coronavirus is selected from reagents that can block or inhibit the binding between the S protein RBD of coronavirus and its cell receptor (e.g., ACE2, such as human ACE2), such as reagents that specifically bind to the S protein RBD of coronavirus or that specifically bind to the cell receptor of coronavirus (e.g., ACE2, such as human ACE2).

In certain embodiments, the reagent is selected from the group consisting of a neutralizing antibody or blocking antibody that specifically binds to the S protein RBD of coronavirus, a polypeptide or protein derived from coronavirus cell receptor (e.g., ACE2) (e.g., a polypeptide or proteins containing ACE2 ectodomain), or a polypeptide or protein derived from the RBD protein or S1 protein of coronavirus (e.g., a polypeptide or protein comprising the full-length sequence of the RBD protein or S 1 protein or an active fragment thereof).

In another aspect, the present invention also relates to a use of the fusion protein of the first aspect, the fusion protein of the second aspect, the multimer of the third aspect, or the kit of the fourth aspect, in the manufacture of a detection reagent for evaluating the activity of a fusion inhibitor against a coronavirus, and/or for screening a fusion inhibitor against a coronavirus.

In certain embodiments, the detection reagent undergoes the method of the fifth aspect to evaluate the activity of the fusion inhibitor against the coronavirus, and/or to screen the fusion inhibitor against the coronavirus. In certain embodiments, the detection reagent is further used for screening for a drug that can prevent and/or treat a coronavirus infection or a disease associated with a coronavirus infection.

In certain embodiments, the coronavirus uses ACE2 (e.g., human ACE2) as a cell receptor. In certain embodiments, the coronavirus is selected from SARS-CoV-2 and/or SARS-CoV-1. In certain embodiments, the coronavirus is SARS-CoV-2.

### Definition of Terms

In the present invention, unless otherwise specified, scientific and technical terms used herein have the meanings commonly understood by those skilled in the art. Moreover, the laboratory procedures of virology, biochemistry, nucleic acid chemistry, immunology, etc. used herein are all routine steps widely used in the corresponding fields. Meanwhile, for a better understanding of the present invention, definitions and explanations of related terms are provided below.

As used herein, "severe acute respiratory syndrome coronavirus 2 (SARS-CoV-2)", formerly known as "novel coronavirus" or "2019-nCov", belongs to its β coronavirus genus, and is an enveloped, single-stranded positive-sense RNA virus. The genome sequence of SARS-CoV-2 is known to those skilled in the art, see, for example, GenBank: MN908947.

As used herein, "severe acute respiratory syndrome coronavirus 1 (SARS-CoV-1)", belonging to its β coronavirus genus, and is an enveloped, single-stranded positive-sense RNA virus. The genome sequence of SARS-CoV-1 is known to those skilled in the art, see, for example, GenBank: AAP13567.1.

As used herein, the term "vector" refers to a nucleic acid delivery vehicle into which a polynucleotide can be inserted. When the vector can express the protein encoded by the inserted polynucleotide, the vector is called an expression vector. The vector can be introduced into a host cell by transformation, transduction or transfection, so that the genetic material elements carried by it can be expressed in the host cell. Vectors are well known to those skilled in the art and include, but are not limited to: plasmid; phagemid; cosmid; artificial chromosome, such as yeast artificial chromosome (YAC), bacterial artificial chromosome (BAC) or P1 derived artificial chromosome (PAC); phage such as λ phage or M13 phage, and animal viruses and so on. Animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40). A vector may contain a variety of elements that control expression, including, but not limited to, promoter sequence, transcription initiation sequence, enhancer sequence, selection element, and reporter gene. Additionally, the vector may also contain an origin of replication site.

As used herein, the term "host cell" refers to a cell that can be used for the introduction of a vector, including, but not limited to, prokaryotic cell such as *E. coli* or *Bacillus subtilis,* fungal cell such as yeast cell or *Aspergillus,* etc., insect cell such as S2 *Drosophila* cell or Sf9, or animal cell such as fibroblast, CHO cell, COS cell, NSO cell, HeLa cell, BHK cell, HEK 293 cells or human cell.

As used herein, the term "identity" refers to the match degree between two polypeptides or between two nucleic acids. When two sequences for comparison have the same monomer sub-unit of base or amino acid at a certain site (e.g., each of two DNA molecules has an adenine at a certain site, or each of two polypeptides has a lysine at a certain site), the two molecules are identical at the site. The percent identity between two sequences is a function of the number of identical sites shared by the two sequences over the total number of sites for comparison x 100. For example, if 6 of 10 sites of two sequences are matched, these two sequences have an identity of 60%. For example, DNA sequences: CTGACT and CAGGTT share an identity of 50% (3 of 6 sites are matched). Generally, the comparison of two sequences is conducted in a manner to produce maximum identity. Such alignment can be conducted by using a computer program such as Align program (DNAstar, Inc.) which is based on the method of Needleman, et al. (J. Mol. Biol. 48:443-453, 1970). The percent identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

The twenty conventional amino acids referred to herein have been written following their conventional usages. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And in the present invention, amino acids are generally represented by one-letter and three-letter abbreviations well known in the art. For example, alanine can be represented by A or Ala.

### Beneficial Effects of the Present Invention

At present, the screening of coronavirus infection inhibitors often requires operations in laboratories with biosafety level 2 or above (or even 3 or higher), and it often requires more than 24-72 hours of culture from infection to actual detection of infection indicators due to the time required for virus infection and replication, requiring a long time to occupy equipment such as cell culture devices, thereby reducing the efficiency.

In order to overcome the technical limitations of the existing system, the present invention has successfully established a novel screening system that can evaluate the effect of inhibitors to block coronavirus infection under virus-free conditions, which comprises a recombinant protein fluorescent probe that mimics coronavirus, and a cell model stably overexpressing human ACE2. Compared with the known technology, the screening system and method established by the present invention have the following obvious advantages: (1) it uses recombinant fluorescent protein-fused probe, to avoid the use of authentic viruses or pseudoviruses, and can be carried out in ordinary laboratories; (2) It is short time-consuming, and only takes 30-60 minutes to perform detection after incubation of probe and cells; (3) no washing is required during the detection process, and fluorescence microscopy or high-content imaging system can be directly used for analysis, which is suitable for high-throughput screening; (4) it has universality for different species of coronaviruses, and HCoV-HKU1, SARS-CoV-1, SARS-CoV-2 and MERS of the human coronavirus β genus as well as bat coronavirus RaTG13 that is highly homologous to SARS-CoV-2 were all detected in the present invention. The screening system and method established by the present invention show its prospect and value in the application of diagnosis, prevention and treatment of coronaviruses (e.g., SARS-CoV-2).

The embodiments of the present invention will be described in detail below with reference to the drawings and examples, but those skilled in the art will understand that the following drawings and examples are only used to illustrate the present invention, rather than limit the scope of the present invention. Various objects and advantageous aspects of the present invention will become apparent to those skilled in the art from the accompanying drawings and the following detailed description of the preferred embodiments.

### Brief Description of the Drawings

FIG. 1 shows the schematic structures of SARS-CoV2 Spike protein and fluorescent protein-fused RBD protein.
FIG. 2 shows the PAGE electrophoresis analysis of SARS-CoV2-RBG.
FIG. 3 shows the PAGE electrophoresis analysis of SARS-CoV2-RBN and SARS-CoV2-RBD recombinant proteins.
FIG. 4 shows the PAGE electrophoresis analysis of SARS-CoV1-RBG.
FIG. 5 shows the PAGE electrophoresis analysis of MERS-RBG.
FIG. 6 shows the PAGE electrophoresis analysis of HKU1-RBG.
FIG. 7 shows the PAGE electrophoresis analysis of RaTG13-RBG.
FIG. 8 shows fluorescence imaging analysis of native electrophoresis of fluorescent protein-fused RBD proteins of different coronaviruses.
FIG. 9 shows the schematic diagram of the structure of SARS-CoV2 spike protein ectodomain trimer.
FIG. 10 shows PAGE electrophoresis analysis of SARS-CoV2-STN, SARS-CoV2-STG, SARS-CoV2-ST recombinant proteins.
FIG. 11 shows the HPLC analysis of SARS-CoV2-STG and SARS-CoV2-STN.
FIG. 12 shows Western blot analysis of cell lines stably expressing human ACE2. Notes: N represents the cell lysate sample of untransfected 293T cell or H1299 cell, AM represents 293T-ACE2iRb3 cell lysate sample, and A represents 293T-ACE2hR cell or H1299-ACE2hR cell.
FIG. 13 shows the time-effect relationship of the binding and uptake of SARS-CoV2-RBG and SARS-CoV2-STG probes by 293T-ACE2iRb3 cells.
FIG. 14 shows the binding and uptake of SARS-CoV2-RBG and SARS-CoV2-STG probes in the H1299-ACE2hR cell model.
FIG. 15 shows the dose-effect relationship of binding and uptake of different protein probes by 293T-ACE2iRb3 cells.
FIG. 16 shows the functional blocking antibody titers in the serum of mice after immunization detected by CSBT and CRBT.
FIG. 17 shows the evaluation by CSBT and CRBT of the blocking effect of recombinant protein inhibitors against SARS-CoV-2.
FIG. 18 shows the affinity determination of 12 different murine monoclonal antibodies to SARS-CoV-2 RBD.
FIG. 19 shows the correlation of the quantitative detections by the three methods: pseudovirus neutralization model, CRBT and CSBT of the neutralization/blocking activity of 12 monoclonal antibodies.

### Sequence Information

The information of the partial sequences involved in the present invention is provided as follows.

| SEQ ID NO | description |
|---|---|
| 1 | SARS-CoV-2-RBD, amino acid sequence |
| 2 | SARS-CoV-2-RBD, nucleic acid sequence |
| 3 | B2M signal peptide, nucleic acid sequence |
| 4 | Green fluorescent protein mGamillus, nucleic acid sequence |
| 5 | Green fluorescent protein mNeonGreen, nucleic acid sequence |
| 6 | Flexible amino acid linker sequence, amino acid sequence |
| 7 | SARS-CoV2-RBG, amino acid sequence |
| 8 | SARS-CoV2-RBN, amino acid sequence |
| 9 | SARS-CoV2-RBG, nucleic acid sequence |
| 10 | SARS-CoV2-RBN, nucleic acid sequence |
| 11 | SARS-CoV2-RBD-His, nucleic acid sequence |
| 12 | SARS-CoV2-RBD-His, amino acid sequence |
| 13 | SARS-CoV1-RBG, amino acid sequence |
| 14 | SARS-CoV1-RBG, nucleic acid sequence |
| 15 | MERS-RBG, amino acid sequence |
| 16 | MERS-RBG, nucleic acid sequence |
| 17 | HKU1-RBG, amino acid sequence |
| 18 | HKU1-RBG, nucleic acid sequence |
| 19 | RaTG13-RBG, amino acid sequence |
| 20 | RaTG13-RBG, nucleic acid sequence |
| 21 | SARS-CoV2-Secd, amino acid sequence |
| 22 | SARS-CoV2-Secd, nucleotide sequence |
| 23 | TFD, nucleotide sequence |
| 24 | SARS-CoV2-STG, nucleotide sequence |
| 25 | SARS-CoV2-STN, nucleotide sequence |
| 26 | SARS-CoV2-STG, amino acid sequence |
| 27 | SARS-CoV2-STN, amino acid sequence |
| 28 | SARS-CoV2-ST, nucleic acid sequence |
| 29 | SARS-CoV2-ST, amino acid sequence |
| 30 | hACE2, nucleic acid sequence |
| 31 | hACE2mRb3, nucleic acid sequence |
| 32 | hACE2mRb3, amino acid sequence |
| 33 | SARS-CoV2-SMG, amino acid sequence |
| 34 | SARS-CoV2-SMG, nucleic acid sequence |
| 35 | SARS-CoV2-S1, amino acid sequence |
| 36 | SARS-CoV2-S1, nucleic acid sequence |
| 37 | B2M signal peptide, amino acid sequence |
| 38 | Green fluorescent protein mGamillus, amino acid sequence |
| 39 | Green fluorescent protein mNeonGreen, amino acid sequence |
| 40 | TFD, amino acid sequence |
| 41 | hACE2, amino acid sequence |
| 42 | Red fluorescent protein mRuby3, amino acid sequence |

### EXAMPLES

The present invention will now be described with reference to the following examples, which are intended to illustrate, but not limit, the present invention.

Unless otherwise specified, the molecular biology experimental methods and immunoassays used in the present invention were performed by basically referring to J. Sambrook et al., Molecular Cloning: A Laboratory Manual, 2nd Edition, Cold Spring Harbor Laboratory Press, 1989, and F. M. Ausubel et al., Refined Molecular Biology Laboratory Manual, 3rd Edition, John Wiley & Sons, Inc., 1995; and the restriction enzymes were used according to the conditions recommended by the product manufacturer. Those skilled in the art appreciate that the examples describe the present invention by way of example and are not intended to limit the scope sought to be protected by the present invention.

### Example 1. Preparation of fluorescent protein-fused probe based on SARS-CoV-2 receptor-binding domain (RBD) by recombinant expression

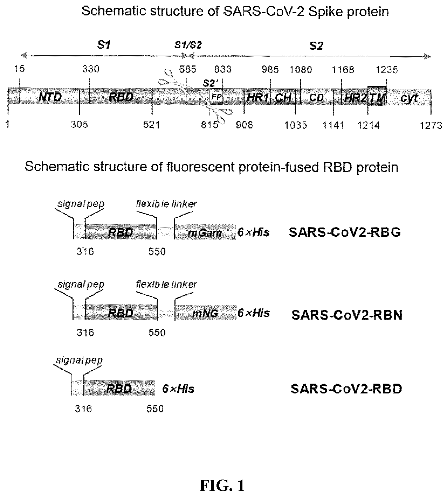

Referring to the complete gene sequence of SARS-CoV2-2 (MN908947.3), the amino acid sequence (SEQ ID NO: 1) of the SARS-CoV2-2 spike protein receptor-binding domain (corresponding to aa316-aa550 of the virus Spike gene) was optimized according to the human codon usage bias to obtain its optimized coding nucleic acid sequence (SEQ ID NO: 2). The nucleic acid sequence of RBD was linked with a signal peptide coding sequence (SEQ ID NO: 3, the leader sequence of human B2M) at its N-terminal, linked with the optimized coding nucleic acid sequence of green fluorescent protein mGamillus (referred to as mGam for short, SEQ ID NO: 4) or mNeonGreen (referred to as mNG for short, SEQ ID NO: 5) at its C-terminal, in which a sequence encoding a flexible linker (SEQ ID NO: 6) was further included between the RBD and green fluorescent protein, and the C-terminal of the fusion polypeptide was linked with a polyhistidine polypeptide (6×His or 8×His) to facilitate affinity chromatography purification, thereby obtaining the fluorescent protein-fused probe based on RBD RBD-mGam (referred to as SARS-CoV2-RBG for short, SEQ ID NO: 7) and RBD-mNG (referred to as SARS-CoV2-RBN for short, SEQ ID NO: 8). The nucleic acid coding sequence of SARS-CoV2-RBG was SEQ ID NO: 9, and the nucleic acid coding sequence of SARS-CoV2-RBN was SEQ ID NO: 10. As a control, we also constructed a SARS-CoV2-RBD protein (with the nucleic acid sequence of SEQ ID NO: 11, the amino acid sequence of SEQ ID NO: 12) that was not fused with the green fluorescent protein at the C-terminal but with the polyhistidine polypeptide (6×His) for purification. The structures of SARS-CoV-2 Spike protein, SARS-CoV2-RBG protein, and SARS-CoV2-RBN protein are shown in FIG. 1.

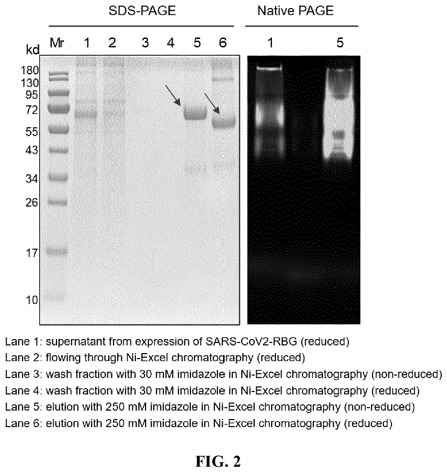

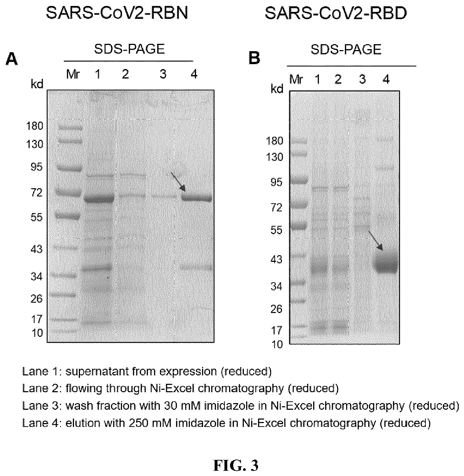

The coding sequences of SARS-CoV2-RBG, SARS-CoV2-RBN and SARS-CoV2-RBD were ligated to a plasmid vector suitable for eukaryotic expression, and the constructed recombinant plasmids were transfected into ExpiCHO cells (purchased from Thermofisher) or other CHO cells for expression and purification, which was performed according to the following steps.
(1) ExpiCHO cells at a cell density of 3×10⁶ were cultured in a triangle shake flask with an appropriate amount of ExpiCHO^{™} Expression Medium (purchased from Thermofisher), and placed in a constant temperature shaker and cultured at 37°C, 8% CO₂, and an appropriate rotation speed for 24 hours, until the cell density reached a density of 6×10⁶.
(2) The SARS-CoV2-RBG and SARS-CoV2-RBN expression plasmids were transfected into cells respectively by using ExpiFectamine CHO Transfection Kit (purchased from Thermofisher), and continuously cultured under the same conditions for 17 to 24 hours, and then added with the culture feed and transfection enhancer as provided in the kit; the cells were changed to a constant temperature shaker and cultured at 32°C, 5% CO₂, and an appropriate rotation speed for 6 days.
(3) After 6 days of culturing, the cell suspension after expression was collected, centrifuged at 12,000 rpm for 30 min at room temperature; the supernatant was collected and dialyzed into PBS, and then filtered through a 0.22 µm filter.
(4) The supernatant sample dialyzed into PBS was purified by medium-pressure Ni-EXCEL chromatography (purchased from GE Healthcare), washed with 15 mM or 30 mM imidazole solution to remove impurities, eluted with 150 mM or 250 mM imidazole solution to obtain the target protein. The obtained target protein was dialyzed into PBS buffer and stored at -20°C.
(5) PAGE analysis was performed on the purified SARS-CoV2-RBG (FIG. 2). Considering the influence of glycosylation, the molecular weight of the target protein was between 65-72kd, and green fluorescent band could be detected by native PAGE. The SDS-PAGE analysis of the purified SARS-CoV2-RBN showed that the size of the target protein was basically the same as that of SARS-CoV2-RBG (FIG. 3A), and the fluorescent signal could be observed with the naked eye. The SDS-PAGE analysis of the purified SARS-CoV2-RBG protein showed that the target protein was about 34-43 kd, which was consistent with expectations (FIG. 3B).

### Example 2. Preparation of fluorescent protein-fused probes based on receptor-binding domain (RBD) of coronavirus such as SARS-CoV-1, MERS-CoV, HKU1-CoV, RaTG13 by recombinant expression

Referring to the viral genome sequences SARS-CoV-1 (AAP13567.1), MERS-CoV (AFS88936.1), HKU1-CoV (YP173238.1) and RaTG13 (MN996532.1) published on Genebank, the RBD sequences of the above four coronaviruses were used to construct fluorescent protein-fused probes including the corresponding RBD and mGamillus by referring to the method in Example 1, wherein:
(1) The recombinant fluorescent protein-fused RBD protein of SARS-CoV-1 is abbreviated as SARS-CoV1-RBG, its amino acid sequence is SEQ ID NO: 13, and its nucleotide coding sequence is SEQ ID NO: 14.
(2) The recombinant fluorescent protein-fused RBD protein of MERS-CoV is abbreviated as MERS-RBG, its amino acid sequence is SEQ ID NO: 15, and its nucleotide coding sequence is SEQ ID NO: 16.
(3) The recombinant fluorescent protein-fused RBD protein of HKU1-CoV is abbreviated as HKU1-RBG, its amino acid sequence is SEQ ID NO: 17, and its nucleotide coding sequence is SEQ ID NO: 18.
(4) The recombinant fluorescent protein-fused RBD protein of RaTG13-CoV is abbreviated as RaTG13-RBG, its amino acid sequence is SEQ ID NO: 19, and its nucleotide coding sequence is SEQ ID NO: 20.

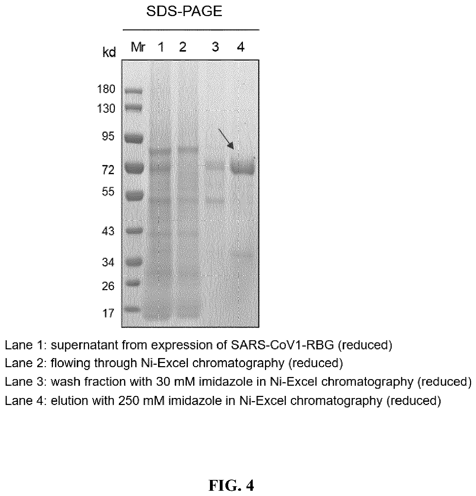

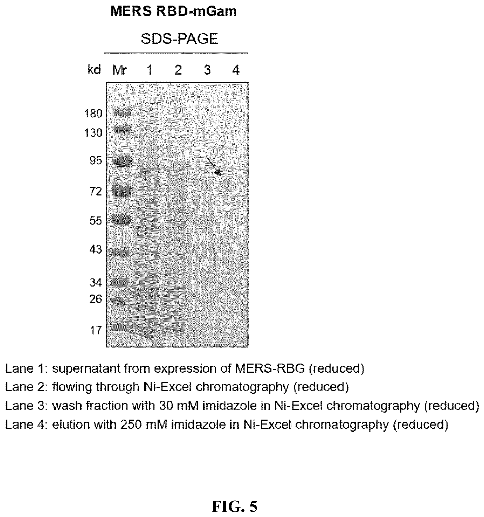

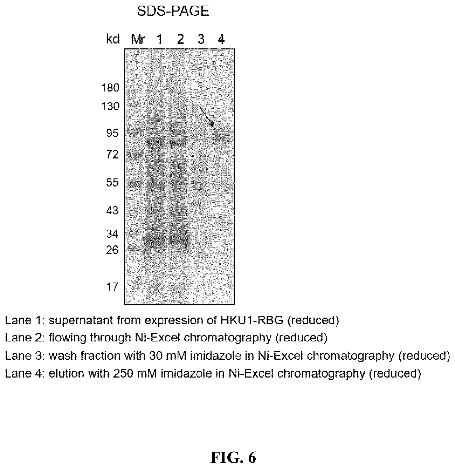

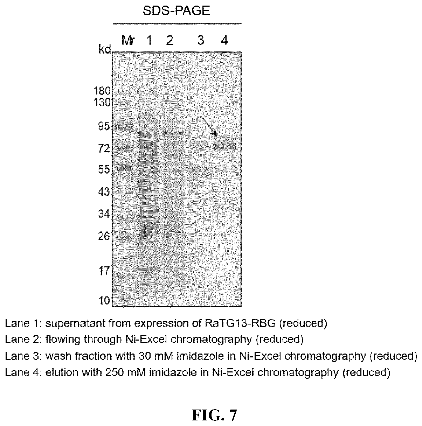

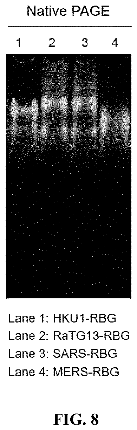

The coding sequences of the above-mentioned 4 kinds of fluorescent protein-fused RBD proteins were constructed into eukaryotic expression vectors, followed by expression in ExpiCHO cells or other CHO cells and purification by affinity chromatography referring to the methods in Example 1, thereby obtaining 4 kinds of fluorescent protein-fused RBD proteins of different coronavirus. These recombinant proteins were analyzed by SDS-PAGE electrophoresis, and the results were shown in FIG. 4 (SARS-CoV1-RBG), FIG. 5 (MERS-RBG), FIG. 6 (HKU1-RBG), and FIG. 7 (RaTG13-RBG). These recombinant proteins were analyzed by denaturing electrophoresis, and green fluorescent protein bands could be observed in a fluorescence imager (FIG. 8).

### Example 3. Preparation of fluorescent protein-fused probes based on SARS-CoV-2 spike protein ectodomain trimer by recombinant expression

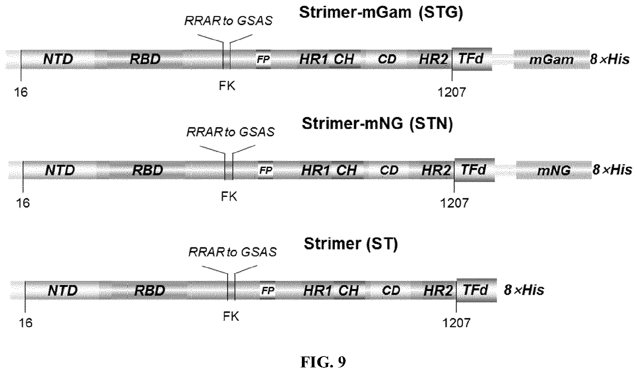

Referring to the full gene sequence of SARS-CoV2-2 (MN908947.3), the full-length amino acid sequence (SEQ ID NO: 21) of the ectodomain of SARS-CoV2-2 spike protein (corresponding to aa16-aa1207 of the virus Spike gene, abbreviated as SARS-CoV2-Secd) was optimized according to human codon usage bias to obtain its optimized coding nucleic acid sequence (SEQ ID NO: 22). The SARS-CoV2-Secd nucleic acid sequence was linked at its N-terminal with a signal peptide coding sequence (SEQ ID NO: 3, the leader sequence of human B2M), linked at its C-terminal with a trimerization domain coding sequence (SEQ ID NO: 23, referred to as TFD for short), and further linked with a coding sequence of flexible linker (SEQ ID NO: 6) and green fluorescent protein mGamillus (referred to as mGam for short, SEQ ID NO: 4) or mNeonGreen (referred to as mNG for short, SEQ ID NO: 5) as well as a polyhistidine polypeptide (6×His or 8×His) to facilitate purification by affinity chromatography, thereby obtaining fluorescent protein-fused probes based on spike protein ectodomain trimer Strimer-mGam (referred to as SARS-CoV2-STG for short, with coding sequence of SEQ ID NO: 24) and Strimer-mNG (referred to as SARS-CoV2-STN for short, with coding sequence of SEQ ID NO: 25). The amino acid sequence of SARS-CoV2-STG is SEQ ID NO: 26, and the amino acid sequence of SARS-CoV2-STN is SEQ ID NO: 27. As a control, we also constructed a SARS-CoV-2 spike protein ectodomain trimer (referred to as SARS-CoV2-ST for short, with nucleic acid sequence of SEQ ID NO: 28, with amino acid sequence of SEQ ID NO: 29) in which the C-terminal was not fused with the green fluorescent protein but with the polyhistidine polypeptide (8×His) for purification. FIG. 9 shows the schematic structures of SARS-CoV2-STG protein, SARS-CoV2-STN protein and SARS-CoV2-ST protein.

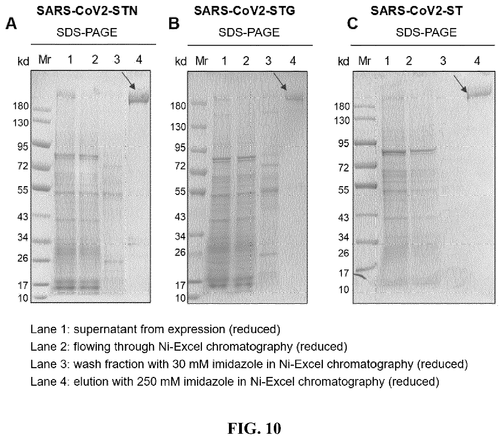

The coding sequences of the above-mentioned three fluorescent protein-fused probes based on SARS-CoV-2 spike protein ectodomain trimer were constructed into eukaryotic expression vectors, following by expression using ExpiCHO cells or other CHO cells and affinity chromatography purification by referring to the method in Example 1, thereby obtaining three spike protein ectodomain trimers of different coronaviruses. These recombinant proteins were analyzed by SDS-PAGE electrophoresis, and the results were shown in FIG. 10A (SARS-CoV2-STN), FIG. 10B (SARS-CoV2 -STG), and FIG. 10C (SARS-CoV2-ST).

In order to accurately measure the molecular weights of the two recombinant proteins of SARS-CoV2-STG and SARS-CoV2-STN, we used fluorescence HPLC for molecular sieve exclusion chromatography analysis. For this analysis, we used TSK-GEL G3000PWxL as the column to detect protein homogeneity (monomer or trimer); the TSK-GEL G3000PW column had an average pore size of 200A, could separate globular proteins with a molecular weight range of 5,000-800,000 Da, and thus met the detection requirements of the target protein.

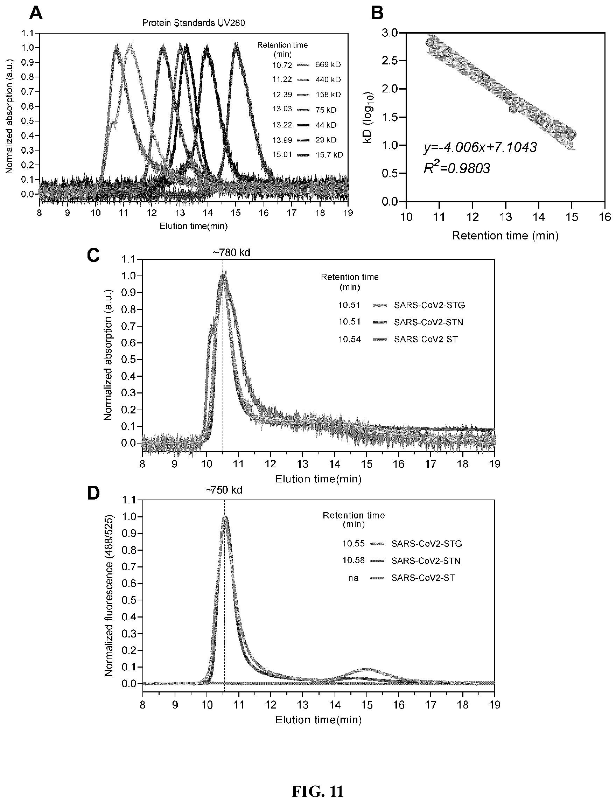

The specific method of HPLC was as follows: (1) chromatographic instrument: Waters type; chromatographic column: TSK-GEL G3000PWxL; mobile phase A: 20mM PBS; program: 0min-30min 100%A; flow rate: 1mL/min; detection wavelength: ultraviolet absorption, 280nm (UV280), fluorescence detection (FI488): 488nm excitation/507nm emission; (2) loading: after equilibrating the pipeline, the column was connected, equilibrated with protein buffer for 60-120 min, and the sample was loaded, which comprised the target protein and standard protein Marker with known molecular weight (Gel Filtration Calibration Kits LMW + HMW, purchased from GE Healthcare Company), 20 µL each; (3) analysis of experimental results: the detection data of UV280 and FI488 with time were exported, and subjected to graphic analysis. By performing linear regression fitting with the retention time of the standard protein marker with known molecular weight on TSK-GEL G3000PW and its actual molecular weight, the formula for calculating the molecular weight of the protein by the retention time was obtained (FIG. 11A and FIG. 11B), thereby the molecular weights of the two recombinant proteins SARS-CoV2-STG and SARS-CoV2-STN were further calculated to be between 750kd (calculated according to FI488, FIG. 11C) and 780kd (calculated according to UV280, FIG. 11D), which were slightly greater than that of the control spike ectodomain trimer (SARS-CoV2-ST) that was not fused with fluorescent protein. The results confirmed that the two proteins were indeed trimers in solution state, and the UV280 peak and the FI488 peak were basically overlapped, indicating that the green fluorescent protein after recombinant fusion still retained its original optical properties.

### Example 4. Construction and identification of cell lines stably expressing human ACE2

Existing studies have confirmed that ACE2 is a cell receptor for coronaviruses such as SARS-CoV1 and SARS-CoV2, and directly interacts with the RBD domain on the viral Spike protein. In order to establish a cell line that is highly reactive with recombinant fluorescent protein-fused probes of coronavirus, we used two methods to construct cell lines stably expressing human ACE2.

### 1. Stable transfection of transposon plasmid

Red fluorescent protein mRuby3 was fused at the C-terminal of the ACE2 gene (NM_021804.1, SEQ ID NO: 30) (connected with a flexible amino acid linker) to obtain the sequence hACE2-mRuby3 (referred to as hACE2mRb3 for short, with nucleic acid sequence of SEQ ID NO: 31); the sequence was cloned into the PiggyBac (PB) transposon vector MIHIP-CMVmie vector constructed in this laboratory (this vector carried a strong CMVmie promoter, which could be ligated to the target gene downstream, and the downstream of the target gene included IRES-driven nuclear localization signal iRFP670 and puromycin resistance gene PuroR for flow sorting and resistance screening) to obtain MIHIP-CMVmie-hACE2mRb3 vector, which could express hACE2mRb3 protein (with amino acid sequence of SEQ ID NO: 32) in cells. The specific method of using MIHIP-CMVmie-hACE2mRb3 to construct the 293T cell line stably expressing human ACE2 was as follows:
(1) 2ml of suspension with 8× 10⁵ 293T cells was coated into a 6-well plate, and placed and cultured in a 37°C, 5% CO₂ incubator overnight (16-24h) until the cell confluence rate reached 70% to 90%, then subjected to transfection.
(2) The MIHIP-CMVmie-hACE2mRb3 plasmid and Super PiggyBac Transposase expression plasmid (purchased from System Biosciences) were co-transfected into cells (according to the mass ratio of 4:1) by using Lipofectamine 3000 transfection reagent (purchased from Thermofisher); after 4 hours of transfection, the medium was changed, the cells were cultured for 24 hours and then passaged to a 10cm cell culture dish; the medium was changed to the medium containing 2 µg/mL puromycin (purchased from InvivoGen) to perform stress screening, and the culture medium for screening the resistance to killing by puromycin was changed every 24 hours.
(3) After 6-7 days of culturing in puromycin-containing culture medium, the cells that were not integrated with the MIHIP-CMVmie-hACE2mRb3 plasmid were almost completely killed by puromycin, and the surviving cells were confirmed to be positive for mRuby3 red fluorescent protein by microscope observation, indicating successful integration. The stably transfected cell line was named as 293T-ACE2iRb3.

### 2. Lentiviral stable transduction

The ACE2 gene (NM_021804.1, SEQ ID NO: 30) was cloned into the lentiviral shuttle vector LvEF1αHRB vector constructed by our laboratory (this vector carried a strong EF1α promoter, and the downstream could be ligated to the target gene, the downstream of the target gene included IRES-driven nuclear localization signal mRuby3 and blasticidin resistance gene BsR for the flow sorting and resistance screening) to obtain LvEF1αHRB-hACE2 vector, and this vector could express hACE2mRb3 protein (with amino acid sequence of SEQ ID NO: 32) in cells. The specific method of using LvEF1αHRB-hACE2 vector to construct 293T cell line and H1299 cell line (human lung cancer cell line) that could stably express human ACE2 was as follows:
(1) 2ml of suspension with 8×10⁵ 293T cells was coated into a 6-well plate, placed and cultured in a 37°C, 5% CO₂ incubator overnight (16-24 hours), and subjected to transfection when the cell confluence rate reached 70% to 90%.
(2) The LvEF1αHRB-hACE2 plasmid and lentiviral packaging plasmids psPAX2 and pMD2.G were co-transfected into cells (according to the mass ratio of 2:1:1) by using Lipofectamine 3000 transfection reagent (purchased from Thermofisher); after 6 hours of transfection, the medium was changed, the culturing was continuously performed for 48 hours under the same conditions, and then the culture supernatant was collected to obtain lentivirus.
(3) 1 mL of the harvested supernatant containing lentivirus was added to H1299 cells and 293T cells cultured in a 6-well plate, added with 1 ul (6ug/mL) of polybrene for increasing infectivity, and subjected to infection under low-speed centrifugation for 1 hour. After the centrifugation was completed, the culturing was continuously performed for 24 hours, and then the culture medium was changed to fresh medium.
(4) After 48 hours, the medium of H1299 cells and 293T cells after lentiviral transduction was changed to medium containing 10 µg/mL blasticidin (purchased from InvivoGen) to perform stress screening, and then the medium was changed every 24 hours.
(5) After 10-14 days of culturing, the cells that were not integrated with the LvEF1αHRB-hACE2 plasmid were almost completely killed by blasticidin, and the surviving cells were confirmed positive for mRuby3 red fluorescent protein by microscope observation, indicating successful integration. The H1299 stably transfected cell line was named as H1299-ACE2hR; and the 293T stably transfected cell line was named as H1299-ACE2hR.

### 3. Identification of cell lines stably expressing human ACE2

In order to verify whether the 293T-ACE2iRb3 cells and the H1299-ACE2hR cells we obtained successfully overexpressed ACE2, western blot was used for detection. The specific detection method was as follows:
(1) The 293T-ACE2iRb3 cells and the H1299-ACE2hR cells at number of 6×10⁵ were coated in 6-well cell culture plates respectively. When the cell confluence rate reached 80% to 90% after 24 hours of adherence, the medium was removed and the cells were washed once with PBS.
(2) 300 µL of cell lysis buffer containing DDM (n-Dodecyl-β-D-maltoside) was added to each well, allowed to stand at 4°C for 1 h for lysis; the lysate was collected in a 1.5mL EP tube, and centrifuged at 12000rpm and 4°C for 10 min; the supernatant was collected into a clean 1.5 mL EP tube, and the lysed samples were subjected to western blot analysis.
(3) ACE2 detection antibody was diluted at 1:1000 (purchased from Sino Biological inc); TMPRSS2 detection antibody was diluted at 1:1000 (purchased from Abcam); HRP-labeled GAPDH antibody (purchased from Proteintech).

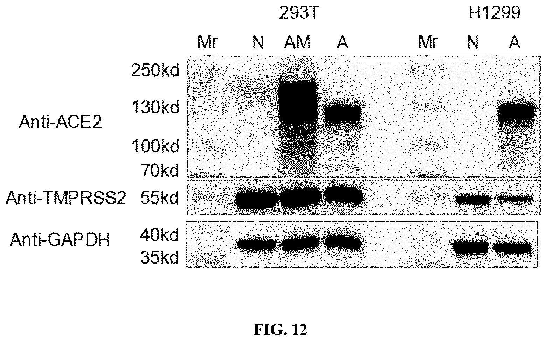

The detection results are shown in FIG. 12, indicating that the three stably transfected cells constructed had successfully overexpressed the coronavirus receptor ACE2.

### Example 5. Binding and internalization of SARS-CoV2-RBG and SARS-CoV2-STG on 293T-ACE2iRb3 cell line

In order to evaluate whether the coronavirus RBG protein and STG protein prepared by recombination can be specifically bound and internalized by the cells expressing ACE2, the two recombinant fluorescent protein-fused probes SARS-CoV2-RBG and SARS-CoV2-STG were evaluated on the 293T-ACE2iRb3 cells constructed in the present invention. The specific method was as follows:
(1) The 293T-ACE2iRb3 cells were coated on black glass bottom microplate at a density of 15,000 cells/well, cultured for 12-24 hours until adherence for later use.
(2) The two recombinant fluorescent protein-fused proteins SARS-CoV2-RBG and SARS-CoV2-STG were diluted by DMEM medium containing 10% fetal bovine serum to the following concentrations: SARS-CoV2-RBG was diluted to 25nM, and SARS-CoV2-STG was diluted to 2.5nM.
(3) 50 µL of the medium was removed from the original cell culture plate, and 50 µL of the dilutions of the two recombinant fluorescent protein-fused proteins SARS-CoV2-RBG and SARS-CoV2-STG were added to the cell culture plate respectively, and incubated for 6 min, 30 min, 60 min and 120 min followed by removing the incubation condition; washing was performed once with PBS, followed by fixing with 0.5% glutaraldehyde; imaging and analysis were performed by using Leica TCS SP8 STED 3X (Leica) laser scanning confocal microscope, in which the imaging fluorescence channels included Ex488/Em510 (green fluorescent protein detection channel, probe signal), Ex561/Em592 (red fluorescent protein detection channel, ACE2), Ex641/Em670 (near-infrared fluorescent protein iRFP670 imaging channel, nucleus).

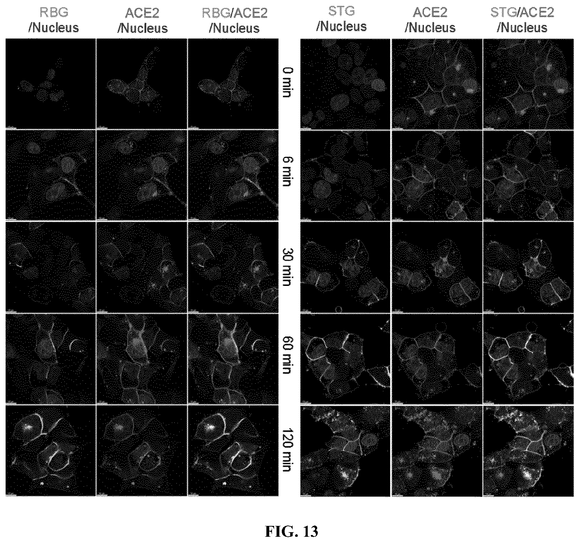

The results shown in FIG. 13 indicated that both SARS-CoV2-RBG and SARS-CoV2-STG could specifically bind to 293T-ACE2iRb3 cells, and its intensity gradually increased over time. In the first 60 min, the detection signals of both probes were mainly concentrated on the cell membrane and showed typical co-localization with ACE2 (red). The 120-minute cell imaging results showed that the detection signals of the two probes in the cytoplasm were significantly increased, and the internalized green signals of SARS-CoV2-STG were significantly more than those of SARS-CoV2-RBG, indicating that the two probes could not only specifically bind to 293T-ACE2iRb3 cells, but also can be internalized and taken up into the cells.

### Example 6. Binding and internalization of SARS-CoV2-RBG and SARS-CoV2-STG on H1299-ACE2hR cell line

In addition to 293T-ACE2iRb3 cells, the two recombinant fluorescent protein-fused probes SARS-CoV2-RBG and SARS-CoV2-STG were also evaluated on the H1299-ACE2hR cells constructed in the present invention. The specific method was as follows:
(1) The H1299-ACE2hR cells were coated on black glass bottom microplate at a density of 10,000 cells/well, and cultured for 12-24 hours until adherence for later use.
(2) The two recombinant fluorescent protein-fused proteins SARS-CoV2-RBG and SARS-CoV2-STG were diluted by DMEM medium containing 10% fetal bovine serum to the following concentrations: SARS-CoV2-RBG was diluted to 25nM, and SARS-CoV2-STG was diluted to 2.5nM.
(3) 50 µL of the medium in the original cell culture plate was removed, and 50 µL of the dilutions of the two recombinant fluorescent protein-fused proteins SARS-CoV2-RBG and SARS-CoV2-STG were added to the cell culture plate respectively, incubated for 12 hours, and then directly subjected to imaging and analysis by using an Opera Phenix confocal high content system without washing.

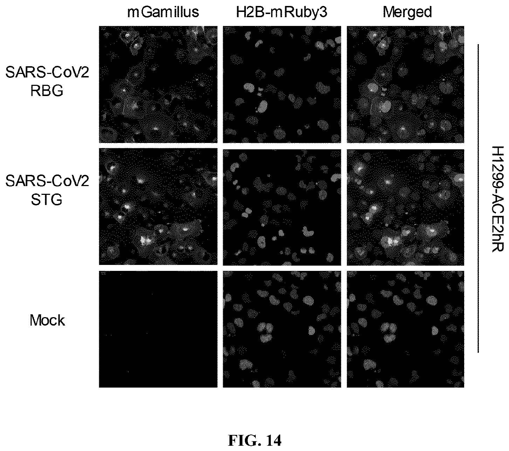

The results in FIG. 14 showed that both SARS-CoV2-RBG and SARS-CoV2-STG could specifically bind to H1299-ACE2hR cells, the green fluorescence signals were found in both cytoplasmic membrane and cytoplasm, and there was obvious fluorescence signal aggregation in the cells, and the intracellular green fluorescence signal of the SARS-CoV2-STG probe was stronger, suggesting that the two probes could not only specifically bind to H1299-ACE2hR cells, but also can be internalized into the cells.

### Example 7. Dose-effect relationship of binding and internalization of recombinant fluorescent protein-fused probes of different coronavirus on 293T-ACE2iRb3 cell line

In order to evaluate the binding and internalization performance of RBG proteins or/and STG proteins of different coronavirus prepared by recombination on cells expressing ACE2, the recombinant fluorescent protein-fused probes of different coronavirus prepared in Examples 1-3 were used, including: SARS-CoV2-RBG, SARS-CoV2-STG, SARS-CoV2-STN, SARS-CoV1-RBG, RaTG13-RBG, MERS-RBG, HKU1-RBG. As a control, a fluorescent protein-fused probe based on non-trimer of SARS-CoV-2 spike protein ectodomain (abbreviated as SARS-CoV2-SMG for short, the amino acid sequence is SEQ ID NO: 33) was obtained by recombination and expression referring to the method of Example 3. Compared with SARS-CoV2-STG, SARS-CoV2-SMG had the removal of trimerization domain TFD at the C-terminal, and the others were the same as SARS-CoV2-STG. In addition, we labeled the RBD recombinant protein (SARS-CoV2-RBD-His, SEQ ID NO: 12, FIG. 3B) and Spike protein ectodomain trimer (SARS-CoV2-ST, SEQ ID NO: 29, FIG. 10C) that were not fused with fluorescent protein with green fluorescent dye DyLight^{™} 488 NHS Ester (purchased from ThermoFisher), the specific method was carried out according to the instructions, with a ratio of 10:1 (molar ratio, dye:protein), to prepare chemical fluorescent dye-labeled SARS-CoV2-RBD488 and SARS-CoV2-ST488 which were used for comparison with the biofluorescent protein-labeled probes as developed in the present invention. The above-mentioned probes were evaluated and verified on the 293T-ACE2iRb3 cells as constructed in the present invention. The specific method was as follows:
(1) The 293T-ACE2iRb3 cells were coated on black glass bottom microplate at a density of 15,000 cells/well, cultured for 12-24 hours until adherence for later use.
(2) A total of 10 recombinant protein probes SARS-CoV2-RBG, SARS-CoV2-STG, SARS-CoV2-STN, SARS-CoV1-RBG, RaTG13-RBG, MERS-RBG, HKU1-RBG, SARS-CoV2-SMG, SARS-CoV2- RBD488 and SARS-CoV2-ST488 were separately diluted by DMEM medium containing 10% fetal bovine serum to different concentrations: 500nM, 250nM, 125nM, 62.5nM, 31.25nM. Among them, five probes including SARS-CoV2-RBG, SARS-CoV2-RBD488, SARS-CoV2-STG, SARS-CoV2-STN and SARS-CoV2-STN were further diluted to lower gradient concentrations: 31.25nM, 15.63nM, 7.81 nM, 3.91 nM, 1.95 nM.
(3) 50 µL of the medium was removed from the original cell culture plate, and 50 µL of the dilutions of above proteins were added to the cell culture plate. After 60 minutes of incubation, imaging and analysis were directly performed without washing by using Opera Phenix confocal high-content system. The imaging fluorescence channels included Ex488/Em510 (green fluorescent protein detection channel, probe signal), Ex561/Em592 (red fluorescent protein detection channel, ACE2), Ex641/Em670 (near-infrared fluorescent protein iRFP670 imaging channel, nucleus), and at least 25 fields of view (confocal mode) were captured by using a 20x or 40x water immersion lens. The data were uploaded to Columbus image management analysis software for quantitative image analysis. The analysis parameters included: number of cells positive for iRFP670 representing nucleus (N, >1000 cells, as required), signal intensity (mean) of cell membrane red fluorescence (ACE2-mRuby3, for interwell normalization), cytoplasmic green fluorescence signal intensity (mean, SD, reflecting the amount of protein probe bound and taken up by cells).
(4) Data processing and statistical analysis: the cytoplasmic green fluorescence signal intensities (mean, SD, N) of the test wells with different doses for each probe were drawn into histograms with Graphpad Prism 8 software for analysis.

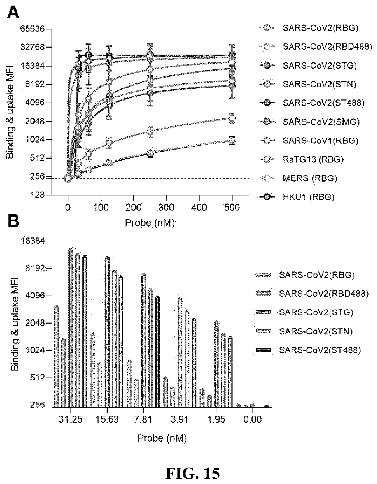

The results in FIG. 15A showed that all 8 probes including SARS-CoV2-RBG, SARS-CoV2-STG, SARS-CoV2-STN, SARS-CoV1-RBG, RaTG13-RBG, SARS-CoV2-SMG, SARS-CoV2-RBD488 and SARS-CoV2-ST488 could specifically bind to 293T-ACE2iRb3 cells in a dose-dependent manner. Since the cell receptors of MERS and HKU1 are not ACE2, the two probes MERS-RBG and HKU1-RBG theoretically have no specific binding to 293T-ACE2iRb3 cells, and the measured signals are regarded as signal background of non-specific binding and uptake. In contrast, the measured signals of MERS-RBG and HKU1-RBG were significantly lower than other probes, and the mean fluorescence signal (MFI) in the cytoplasm was still lower than 1000 even at the highest concentration of 500 nM. Among other probes, SARS-CoV2-STG, SARS-CoV2-STN, SARS-CoV2-ST488 showed the strongest signals, which were significantly stronger than those of the non-trimer control probe SARS-CoV2-SMG and other RBD-based probes; among RBD-based probes, SARS-CoV2-RBG showed a stronger signal than SARS-CoV2-STN, SARS-CoV2-RBD488 and SARS-CoV1-RBG. Although specific signal of binding and uptake were also detected for RaTG13-RBG, it was significantly weaker than that of SARS-CoV2-RBG and SARS-CoV1-RBG, suggesting that the binding of the spike RBD of RaTG13 virus to human ACE2 was significantly weaker than that of SARS-CoV-2 and SARS-CoV-1. In the detection with lower concentrations (FIG. 15B), it could be seen that SARS-CoV2-STG, SARS-CoV2-STN, SARS-CoV2-ST488 that were based on spike protein ectodomain trimer showed significantly stronger detection signal intensities, in which the fluorescence signal intensity of SARS-CoV2-STG at a dose of 2-4 nM could reach the fluorescence signal intensity of SARS-CoV2-RBG at a dose of 31.25 nM. Among the three probes SARS-CoV2-STG, SARS-CoV2-STN and SARS-CoV2-ST488, SARS-CoV2-STG showed stronger signals than SARS-CoV2-STN which showed slightly stronger signals than SARS-CoV2-ST488, indicating that the chemical fluorescent dye-labeled spike protein ectodomain trimer could have a weakened binding ability because the non-specific labeling blocked a part of the binding site for the protein and receptor, and similar phenomenon could also be observed when comparing SARS-CoV2-RBG and SARS-CoV2-RBD488. The above results showed that the fluorescent protein-fused probes developed in the present invention had higher biological activity than the probe prepared by the chemical fluorescent dye labeling method.

### Example 8. Application of the recombinant fluorescent protein-fused probes SARS-CoV2-RBG and SARS-CoV2-STG in evaluation of neutralization antibody in immune serum against SARS-CoV-2 antigen

Based on the specific binding and uptake of recombinant fluorescent protein-fused probes SARS-CoV2-STG and SARS-CoV2-RBG by 293T-ACE2iRb3, we established a cell-based SARS-CoV2-STG function blocking test (cell based spike function blocking test, CSBT) and a cell-based SARS-CoV2-RBG function blocking test (cell based RBD function blocking test, CRBT), respectively. In this example, the two methods were used to detect function blocking antibody titers in the serum of mice immunized with different SARS-CoV-2 membrane protein antigens (recombinant RBD, recombinant S1 subunit, and recombinant S2 subunit), and the results were compared with the neutralizing antibody titers determined by the pseudovirus neutralization assay of SARS-CoV-2 based on lentiviral vector.

### 8.1 Preparation of mouse immune serum against recombinant RBD, recombinant S1 subunit and recombinant S2 subunit

The preparation of SARS-CoV-2 RBD antigen was carried out with reference to the method of Example 3 (SARS-CoV2-RBD-His, SEQ ID NO: 12, FIG. 3B). SARS-CoV-2 recombinant S1 antigen (amino acid sequence SEQ ID NO: 35, nucleic acid sequence SEQ ID NO: 36) was expressed and purified with reference to the method of Example 3. Recombinant S2 antigen was purchased from Beijing Yiqiao Shenzhou Company. The three antigens were mixed with aluminum adjuvant for immunization. BALB/c mice aged 6-8 weeks were immunized with at week 0, week 2 and week 4 with a dose of 10 µg, and the serum samples were collected for detection 1 week after the triple immunization. 3 BALB/c mice were immunized with S1 antigen, 3 BALB/c mice were immunized with S2 antigen, and 5 BALB/c mice were immunized with RBD antigen. Serum samples were collected after the above immunization, and subjected to heat treatment at 56°C for 30 minutes to inactivate complement for later use.

### 8.2 Determination of neutralization titers based on pseudovirus neutralization assay of mouse immune serum against recombinant RBD, recombinant S1 subunit and recombinant S2 subunit

The SARS-CoV-2 pseudovirus neutralization assay was constructed by the pseudovirus system based on lentiviral vector. The specific steps were as follows:
(1) 293T cells were thawed and cultured in DMEM medium containing 10% fetal bovine serum to logarithmic growth phase; 7×10⁶ of 293T cells were inoculated on a 10cm cell culture plate, placed and cultured in a 5% CO₂ cell incubator at 37°C for 12h, and subjected to transfection when the cell confluence reached 95-99%.
(2) The 293T cells were co-transfected with plasmids psPAX2, pLvEF1αmNGNL and EIRBsMie-SARS2-SFL using Lipofectamine^{™} 3000 (ThermoFisher Scientific company) according to the instructions of the kit, to package SARS-CoV-2 pseudovirus (referred to as SARS-CoV2-LvPP). After 6 hours of transfection, the packaging medium was removed and replaced. After 24 hours of transfection, the pseudoviruses were collected for the first time, and all cell supernatants were collected and stored at 4°C. By replacement with pre-warmed fresh medium, culturing was continued in a 37°C, 5% CO₂ cell incubator for 24 hours. 48 hours after transfection, the pseudoviruses were collected for the second time, and the collected cell supernatants were mixed with the supernatants collected for the first time. Centrifugation was performed at 2000 rpm for 30 minutes, and the supernatant was filtered with a 0.45 µm pore size filter for later use.
(3) The H1299ACE2hR cells in logarithmic growth phase were inoculated in a 96-well plate at a density of 1.2×10⁴/100µL/well, and cultured overnight in a 37°C, 5% CO₂ cell incubator. 60 µL of the inactivated immune serum dilutions with different dilution degrees (with DMEM medium containing 10% fetal bovine serum as the diluent) was mixed with equal amount of 1.0×10⁵ TU/mL SARS-CoV2-LvPP, and incubated at 37°C for 1 hour to allow the complete binding between the antibody and the pseudovirus.
(4) 100 µL of the incubation solution of virus and serum prepared in step (3) was added to the plated H1299ACE2hR cells, and incubated in a 37°C, 5% CO₂ cell incubator for infection.
(5) After 36-48 hours of infection, the fluorescence imaging of the infected cells was performed by a spinning-disk confocal high-content imaging system (Opera phenix or Operetta CLS, purchased from Perkinelmer) (20x water immersion lens, 25 fields of view were captured). Columbus image management analysis software was used to quantitatively analyze the obtained fluorescent images to calculate the number of cells positive for green fluorescent protein (mNeonGreen) in each well.
(6) The infection inhibition rate of each well was calculated by comparing with the average number of positive cells in the infection control wells without serum. The calculation formula is as follows: (number of green fluorescence positive cells in positive control well - number of green fluorescence positive cells in test well)/number of green fluorescence positive cells in positive control well × 100%. The inhibition rates of the wells added with serum of different dilution degrees were calculated, and the inhibition curve was drawn by using the Graphpad Prism 8 software, and the 4-parameter curve fitting model was used to calculate the maximum dilution factor (ID50) to achieve an effective infection of 50% (where the positive control infection was set as 100%), which was used to quantitatively indicate the level of neutralizing antibody titer.

### 8.3 Detection of function blocking antibody titers in serum of immunized mice using CSBT and CRBT

(1) The 293T-ACE2iRb3 cells were plated on a black glass bottom microplate at a density of 15,000 cells/well, cultured for 12-24 hours until adherence for later use.
(2) For CSBT detection, the SARS-CoV2-STG probe was diluted to an appropriate concentration (2-4 nM) and mixed with mouse serum dilutions of different dilution factors; for CRBT detection, the SARS-CoV2-RBG probe was diluted to an appropriate concentration (20-30 nM) and mixed with mouse serum dilutions of different dilution factors. 50 µL of medium was removed from the original cell culture plate, and 50 µL of the prepared mixture was added to the cell culture plate, and incubated at 37°C.
(3) 50 µL of medium was removed from the original cell culture plate, and 50 µL of the above protein dilution was added to the cell culture plate. After 60 minutes of incubation, imaging analysis was directly performed without washing by using Opera Phenix confocal high-content system, in which imaging fluorescence channels included Ex488/Em510 (green fluorescent protein detection channel, probe signal), Ex561/Em592 (red fluorescent protein detection channel, ACE2), Ex641/Em670 (near-infrared fluorescent protein iRFP670 imaging channel, nucleus), and at least 25 fields of view were captured by 20x or 40x water immersion lens (confocal mode). The data were uploaded to Columbus image management analysis software for quantitative image analysis. Analysis parameters include: number of cells positive for iRFP670 representing nucleus (N, >1000 cells, as required), signal intensity (mean) of cell membrane red fluorescence (ACE2-mRuby3, for interwell normalization), cytoplasmic green fluorescence signal intensity (mean, SD, reflecting the amount of protein probe bound and taken up by cells).
(4) Data processing and statistical analysis: inhibition rate = the differences between the average intensities of cytoplasmic green fluorescence signals in different test wells of SARS-CoV2-STG and SARS-CoV2-RBG probes and that of the positive control wells/the average fluorescence intensity of the positive control wells × 100%, the Graphpad Prism 8 software was used to draw the inhibition curve, and the 4-parameter curve fitting model was used to calculate the ID50.

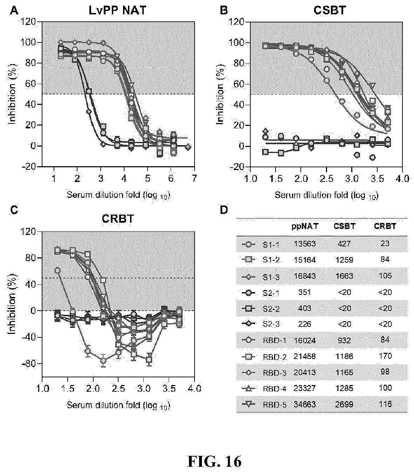

As shown in FIG. 16, the immune serum against S1 antigen and RBD antigen showed detectable higher titers of neutralizing blocking antibodies in all three assays: SARS-CoV2-LvPP (FIG. 16A), CSBT (FIG. 16B) and CRBT (FIG. 16C); the immune serum against S2 antigen showed no detectable blocking antibodies in the CSBT and CRBT assays, while its neutralizing activity could be measured in the SARS-CoV2-LvPP pseudovirus neutralization assay, but the result was 62 times lower than the immune serum against S1 antigen or RBD antigen. Overall, the neutralization titers measured using the SARS-CoV2-LvPP pseudovirus neutralization assay had a better correlation with the CSBT titers (R²=0.973), and a significant correlation with the CRBT titers (R²=0.725). The above results suggested that the CSBT assay could be used as a surrogate indicator of immune serum neutralization titers, and the CSBT assay titers were highly linearly correlated with the NAT titers of pseudovirus neutralization assay. Compared with the pseudovirus neutralization assay which takes 24-48 hours, CSBT only needs 1-1.5 hours for detection, and the efficiency is greatly improved.

### Example 9. Evaluation of SARS-CoV-2 recombinant protein inhibitor based on SARS-CoV2-RBG and SARS-CoV2-STG recombinant fluorescent protein-fused probes

In order to evaluate whether the CSBT and CRBT methods established in the present invention can be used to evaluate the recombinant protein infection inhibitor against SARS-CoV-2, we used the detection method of Example 8 to evaluate the recombinant protein inhibitors ACE2-Ig protein, RBD protein of SARS-CoV-2, S1 protein of SARS-CoV-2, and cross-binding antibody CR3022 of SARS-CoV-1/2 that might have infection inhibitory effect as reported in the literatures. ACE2-Ig is a recombinant protein prepared by fusing the extra-membrane region of receptor protein ACE2 with the Fc of human IgG1, and has the function of binding in a competing manner to the ACE2 receptor on the cell to which SARS-CoV-2 binds (Changhai Lei, Wenyan Fu, Kewen Qian, et al. Potent neutralization of 2019 novel coronavirus by recombinant ACE2-Ig. bioRxiv preprint doi: https://doi.org/10.1101/2020.02.01.929976). The RBD and S1 proteins of SARS-CoV-2 have the function of competing with probes for binding to cell receptors. CR3022 is a recombinant human monoclonal antibody isolated from a patient recovered from the infection of SARS-CoV-1 (Ter Meulen J, van den Brink EN, Poon LL et al. Human monoclonal antibody combination against SARS coronavirus: synergy and coverage of escape mutants. PLoS Med. 2006 Jul;3(7):e237.), which binds to the conserved epitope of SARS-CoV-1/2, but it only has a certain neutralization effect on SARS-CoV-1, while has no neutralization effect on SARS-CoV-2 (Yuan M, Wu NC, Zhu X, et al. A highly conserved cryptic epitope in the receptor-binding domains of SARS-CoV-2 and SARS-CoV. Science. 2020 Apr 3. pii: eabb7269.).

We performed the detection of the above 4 recombinant proteins by using the CSBT and CRBT detection methods in Example 8, with a total of 9 test concentration gradients of 500nM, 250nM, 125nM, 62.5nM, 31.3nM, 15.6nM, 7.8nM, 3.9nM, 2.0nM. The cytoplasmic green fluorescence signal intensity of each test well was calculated, and the inhibition rate (%) was calculated by comparing with the positive control well.

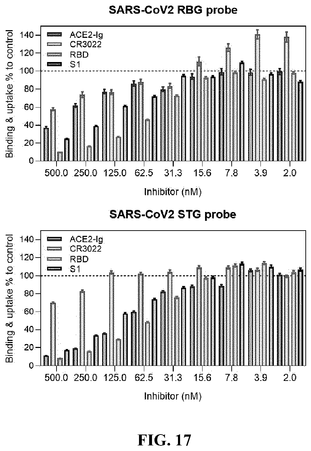

The results were shown in FIG. 17. For the results obtained by SARS-CoV2-RBG probe (FIG. 17A), the inhibitory effects of RBD protein and S1 protein were better than that of ACE2-Ig, and a certain extent of inhibitory effect of the CR3022 antibody could only be observed under extremely high concentrations, but the inhibition rate did not reach >50% even at the highest test concentration of 500 nM. For the results obtained by SARS-CoV2-STG probe (FIG. 17B), the inhibitory effects of RBD protein, S1 protein and ACE2-Ig were roughly equivalent, but RBD protein was still better. Likewise, no significant inhibition effect of CR3022 was observed in the CSBT assay. The above results are highly consistent with the expected results reported in the literatures, indicating that the CSBT and CRBT methods established in the present invention can be used to evaluate the efficacy of recombinant protein inhibitors of SARS-CoV-2.

### Example 10. Evaluation of infection blocking effect of monoclonal antibody against SARS-CoV-2 based on SARS-CoV2-RBG and SARS-CoV2-STG recombinant fluorescent protein-fused probes

In order to test the application of the CRBT and CSBT detection methods based on the SARS-CoV2-RBG and SARS-CoV2-STG recombinant fluorescent protein-fused probes established in the present invention in the evaluation of the infection blocking effect of anti-SARS-CoV-2 monoclonal antibodies, we prepared 12 monoclonal antibodies by immunizing mice with SARS-CoV2-RBD recombinant protein, and performed evaluation of these antibodies. The specific method was as follows:
10.1 Preparation of mouse monoclonal antibodies against SARS-CoV-2 receptor-binding region
10.1.1 The recombinantly expressed SARS-CoV2-RBD antigen as in Example 1 was used as the immunogen, the recombinant protein was diluted to 0.2 mg/mL, mixed with an equal volume of Freund's adjuvant and thoroughly mixed and emulsified. BALB/c female mice aged 6-8 weeks were immunized by subcutaneous multipoint injections on bilateral groin. The injection volume was 300 µL/mouse/time. About 200 µL of orbital venous blood was collected before each immunization for titer determination, and booster immunization was performed once every 2 weeks after the initial immunization, and cell fusion was performed 4 weeks later.
10.1.2 The recombinantly expressed SARS-CoV2-RBD antigen as in Example 1 was used as the immunogen, the recombinant protein was diluted to 0.2 mg/mL, mixed with an equal volume of Freund's adjuvant and thoroughly mixed and emulsified. BALB/c female mice aged 6-8 weeks were immunized by subcutaneous multipoint injections on bilateral groin. The injection volume was 300 µL/mouse/time. About 200 µL of orbital venous blood was collected before each immunization for titer determination, and booster immunization was performed once every 2 weeks after the initial immunization, and cell fusion was performed after 4 weeks.
10.1.3 The splenocytes of the immunized mice were PEG-fused with mouse myeloma cells (SP2/0) to obtain monoclonal antibody hybridoma cells. The unfused myeloma cells and mouse splenocytes died in RPMI1640-HAT screening medium, while the successfully fused hybridoma cells survived.
10.1.4 Detection and screening of hybridoma cells: The recombinant SARS-CoV-2 RBD protein prepared in Example 1 was coated on an ELISA microplate, after blocking, the fusion cell culture supernatant was added, and reacted at 37°C for 1 hour. Then HRP-labeled goat anti-mouse secondary antibody was added, reacted at 37°C for half an hour, and then subjected to color development, and positive clones with high reactivity were picked according to the OD value detected by ELISA.
10.1.5 After obtaining the positive hybridoma cells, monoclonalization was performed to obtain a total of 12 monoclonal antibody cell lines, including 12H8, 14D2, 15A9, 16B12, 21F7, 23B1, 34B4, 3C8, 53G2, 5F3, 60A11 and 8H6.
10.1.6 After the 12 monoclonal antibody cell lines were cultured, ascites was prepared and purified by Protein A affinity chromatography to obtain purified monoclonal antibodies, and BCA was used to detect the protein concentrations of the purified monoclonal antibodies.
10.2 Determination of affinity of murine monoclonal antibodies to SARS-CoV2-RBD

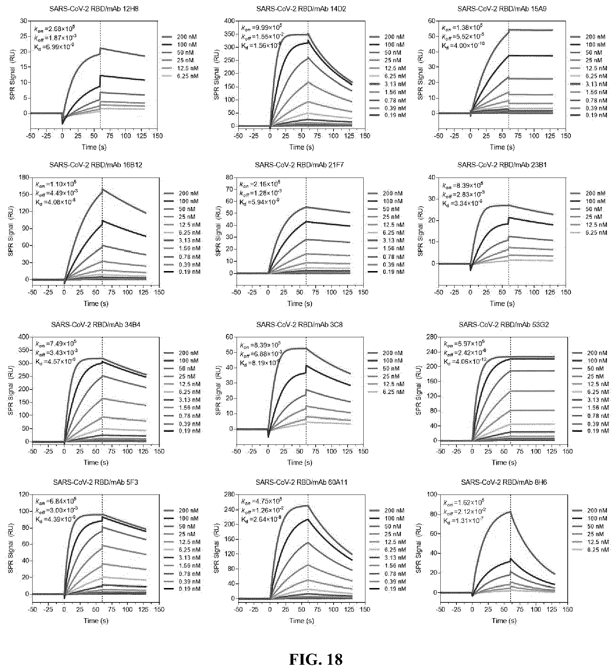

The affinity of 12 mouse monoclonal antibodies to SARS-CoV2-RBD protein was detected by surface plasmon resonance (SPR) on Biacore 8000 (GE Company). The results shown in FIG. 18 showed that all 12 monoclonal antibodies could bind to SARS-CoV2-RBD protein with high affinity, in which the affinity was 6.99 nM for 12H8, 15.6 nM for 14D2, 0.4 nM for 15A9, 40.8 nM for 16B12, 5.94 nM for 21F7, 3.34 nM for 23B1, 4.57 nM for 34B4, 8.19 nM for 3C8, 0.004 nM for 53G2, 4.39 nM for 5F3, 26.4 nM for 60A11, and 131 nM for 8H6.

10.3 Determination of neutralizing activity of mouse monoclonal antibodies on SARS-CoV-2 pseudovirus model and determination of blocking activities by CSBT and CRBT

The SARS-CoV-2 pseudovirus (SARS-CoV2 LvPP) was prepared using the method of Example 8, and the neutralization titer was detected for 12 monoclonal antibodies by SARS-CoV2 LvPP, in which the maximum initial concentration of each monoclonal antibody was set to 500 nM, and at least 9 gradients were achieved by 2-fold dilution. 60 µL of the antibody dilutions of different concentrations were mixed with equal amount of 1.0×10⁵ TU/mL SARS-CoV2-LvPP, and incubated at 37°C for 1 hour to allow the complete binding between the antibody and the pseudovirus. Then, 100 µL of the virus-antibody mixture was added to the H1299ACE2hR cells that had been plated and cultured in a 96-well cell culture plate, and cultured in a 37°C, 5% CO₂ cell incubator for infection. After 36-48 hours of infection, the cells after the infection were subjected to fluorescence imaging using a spinning-disk confocal high-content imaging system (Opera phenix or Operetta CLS, purchased from Perkinelmer) (20x water immersion lens, 25 fields of view were captured). Columbus image management analysis software was used to quantitatively analyze the obtained fluorescent images to calculate the number of cells positive for green fluorescent protein (mNeonGreen) in each well. By comparing to the average number of positive cells in the infection control wells without antibody, the infection inhibition rate of each well was calculated. The calculation formula is as follows: (number of green fluorescence positive cells in positive control well - number of green fluorescence positive cells in test well)/number of green fluorescence positive cells in positive control well × 100%. The inhibition rates of the antibodies at different doses were calculated, the inhibition curve was drawn by the Graphpad Prism 8 software, and the 4-parameter curve fitting model was used to calculate the half maximum inhibitory concentration (IC50) and 90% maximum inhibitory concentration IC90. The statistics of the results are shown in Table 1.

In addition to the SARS-CoV2 LvPP pseudovirus neutralization assay, we also performed CRBT and CSBT titer assays on these monoclonal antibodies using the method of Example 8. The highest initial concentration of each antibody was set at 500 nM, and at least 9 gradients were achieved by 2-fold dilution. The inhibition rates (%) = the difference between the average intensity of cytoplasmic green fluorescence signals in different test wells of SARS-CoV2-STG and SARS-CoV2-RBG probes and that of the positive control wells/the average fluorescence intensity of positive control wells × 100%, the Graphpad Prism 8 software was used to draw the inhibition curve, and the 4-parameter curve fitting model was used to calculate the IC50. The statistics of the results are shown in Table 1.

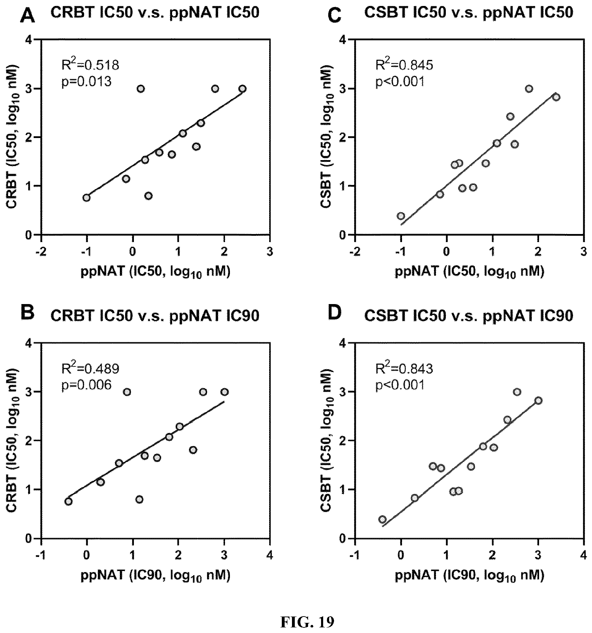

Comparing the neutralizing and blocking activities of the antibodies determined by SARS-CoV2 LvPP NAT, CRBT and CSBT, it can be found that among the 12 antibodies, 12H8, 14D2, 15A9, 21F7, 34B4, 3C8, 5F3 and 60A11 could block the interaction between SARS-CoV2-RBG and ACE2 to different extents, while 23B1, 53G2 and 8H6 could not directly block the interaction between the two. Regression analysis was performed on the values measured by the three methods. The results of FIG. 19 showed that the antibody IC50 values determined by CSBT showed the best correlation with the IC50 values and IC90 values determined by the LvPP NAT method, with R² reaching to 0.845 and 0.843, respectively; meanwhile, the antibody IC50 values determined by CRBT also showed a certain degree of positive correlation with the IC50 values and IC90 values determined by LvPP NAT method. The above results prove that the two novel methods established by the present invention can be used for the evaluation of the neutralization and blocking effects of monoclonal antibodies.

**Table 1. Neutralizing activity of 12 RBD monoclonal antibodies on SARS-CoV2 LvPP pseudovirus model**

| Name of monoclonal antibody | SARS-CoV2 LvPP NAT | | CRBT | CSBT |
|---|---|---|---|---|
| | IC50 (nM) | IC90 (nM) | IC50 (nM) | IC50 (nM) |
| 12H8 | 0.71 | 2.00 | 14.1 | 6.72 |
| 14D2 | 24.4 | 210.1 | 64.7 | 267.8 |
| 15A9 | 30.3 | 106.0 | 196.7 | 72.1 |
| 16B12 | 12.5 | 62.6 | 120.2 | 76.1 |
| 21F7 | 1.86 | 4.99 | 34.6 | 29.7 |
| 23B1 | 246.9 | 1000 | 1000 | 666.4 |
| 34B4 | 2.20 | 14.0 | 6.32 | 9.01 |
| 3C8 | 0.10 | 0.40 | 5.77 | 2.46 |
| 53G2 | 1.50 | 7.49 | 1000 | 27.3 |
| 5F3 | 7.14 | 33.9 | 44.6 | 29.2 |
| 60A11 | 3.81 | 18.3 | 48.8 | 9.36 |
| 8H6 | 62.2 | 341.3 | 1000 | 1000 |

Although specific embodiments of the present invention have been described in detail, those skilled in the art will appreciate that various modifications and changes can be made to the details in light of all the teachings that have been published, and that these changes are all within the scope of the present invention. The full division of the present invention is given by the appended claims and any equivalents thereof.

## Claims

1. A fusion protein, which comprises a S protein receptor-binding domain (RBD) of a coronavirus, and a fluorescent protein.

2. The fusion protein according to claim 1, wherein the coronavirus is selected from the group consisting of SARS-CoV-2, SARS-CoV-1, MERS-CoV, HKU1-CoV or RaTG13.

3. The fusion protein according to claim 1 or 2, wherein the fusion protein comprises the S protein receptor-binding domain and the fluorescent protein from the N-terminal to the C-terminal.

4. The fusion protein according to any one of claims 1 to 3, wherein the S protein receptor-binding domain (RBD) of SARS-CoV-2 comprises: (i) a sequence set forth in SEQ ID NO: 1, or (ii) a sequence having a sequence identity of at least 80%, at least 85%, at least 90%, at least 91%, at least 92%, at least 93%, at least 94%, at least 95%, at least 96%, at least 97%, at least 98%, or at least 99% as compared to SEQ ID NO: 1, or (iii) a sequence having a substitution, deletion or addition of one or several amino acids (e.g., a substitution, deletion or addition of 1, 2, 3, 4 or 5 amino acids) as compared to SEQ ID NO: 1;
for example, the S protein receptor-binding domain (RBD) of SARS-CoV-2 is encoded by a sequence set forth in SEQ ID NO: 2.

5. The fusion protein according to any one of claims 1 to 3, wherein the S protein receptor-binding domain (RBD) of SARS-CoV-1 comprises: (i) a sequence consisting of amino acid residues at the positions 21 to 256 of SEQ ID NO: 13, or (ii) a sequence having a sequence identity of at least 80%, at least 85%, at least 90%, at least 91%, at least 92%, at least 93%, at least 94%, at least 95%, at least 96%, at least 97%, at least 98%, at least 99% or 100% as compared to the sequence of (i), or (iii) a sequence having a substitution, deletion or addition of one or several amino acids (e.g., a substitution, deletion or addition of 1, 2, 3, 4 or 5 amino acids) as compared to the sequence of (i).

6. The fusion protein according to any one of claims 1 to 3, wherein the S protein receptor-binding domain (RBD) of MERS-CoV comprises: (i) a sequence consisting of amino acid residues at the positions 21 to 274 of SEQ ID NO: 15, or (ii) a sequence having a sequence identity of at least 80%, at least 85%, at least 90%, at least 91%, at least 92%, at least 93%, at least 94%, at least 95%, at least 96%, at least 97%, at least 98%, at least 99% or 100% as compared to the sequence of (i), or (iii) a sequence having a substitution, deletion or addition of one or several amino acids (e.g., a substitution, deletion or addition of 1, 2, 3, 4 or 5 amino acids) as compared to the sequence of (i).

7. The fusion protein according to any one of claims 1 to 3, wherein the S protein receptor-binding domain (RBD) of HKU1-CoV comprises: (i) a sequence consisting of amino acid residues at the positions 21 to 349 of SEQ ID NO: 17, or (ii) a sequence having a sequence identity of at least 80%, at least 85%, at least 90%, at least 91%, at least 92%, at least 93%, at least 94%, at least 95%, at least 96%, at least 97%, at least 98%, at least 99% or 100% as compared to the sequence of (i), or (iii) a sequence having a substitution, deletion or addition of one or several amino acids (e.g., a substitution, deletion or addition of 1, 2, 3, 4 or 5 amino acids) as compared to the sequence of (i).

8. The fusion protein according to any one of claims 1 to 3, wherein the S protein receptor-binding domain (RBD) of RaTG13 comprises: (i) a sequence consisting of amino acid residues at the positions 21 to 257 of SEQ ID NO: 19, or (ii) a sequence having a sequence identity of at least 80%, at least 85%, at least 90%, at least 91%, at least 92%, at least 93%, at least 94%, at least 95%, at least 96%, at least 97%, at least 98%, at least 99% or 100% as compared to the sequence of (i), or (iii) a sequence having a substitution, deletion or addition of one or several amino acids (e.g., a substitution, deletion or addition of 1, 2, 3, 4 or 5 amino acids) as compared to the sequence of (i).

9. The fusion protein according to any one of claims 1 to 8, wherein the S protein receptor-binding domain and the fluorescent protein are optionally linked by a peptide linker (e.g., a flexible peptide linker).

10. The fusion protein according to claim 9, wherein the peptide linker comprises 1 to 15 contiguous amino acid residues that are identical or different and selected from glycine and serine;
for example, the peptide linker is (GₘS)ₙ, wherein m is an integer selected from 1 to 4 and n is an integer selected from 1 to 3; for example, the peptide linker comprises a sequence set forth in SEQ ID NO:6.

11. The fusion protein according to any one of claims 1 to 10, wherein the fluorescent protein is selected from the group consisting of green fluorescent protein, blue fluorescent protein, cyan fluorescent protein, yellow fluorescent protein, orange or red fluorescent protein, near-infrared fluorescent protein, or long Stoke shift fluorescent protein;
for example, the fluorescent protein is a green fluorescent protein, such as mGamillus, mNeonGreen, EGFP, mClover, UnaG, TurboGFP, TagGFP, Venus, EYFP, RFP, iRFP670, mBeRFP, CyOFP1;
for example, the fluorescent protein comprises a sequence set forth in SEQ ID NO: 38 or 39.

12. The fusion protein according to any one of claims 1 to 11, comprising an amino acid sequence selected from the group consisting of:
(1) an amino acid sequence consisting of amino acid residues at the positions 21 to 516 of the sequence set forth in SEQ ID NO: 7;
(2) an amino acid sequence consisting of amino acid residues at the positions 21 to 514 of the sequence set forth in SEQ ID NO: 8;
(3) an amino acid sequence consisting of amino acid residues at the positions 21 to 515 of the sequence set forth in SEQ ID NO: 13;
(4) an amino acid sequence consisting of amino acid residues at the positions 21 to 533 of the sequence set forth in SEQ ID NO: 15;
(5) an amino acid sequence consisting of amino acid residues at the positions 21 to 608 of the sequence set forth in SEQ ID NO: 17; or
(6) an amino acid sequence consisting of amino acid residues at the positions 21 to 516 of the sequence set forth in SEQ ID NO: 19.

13. The fusion protein according to any one of claims 1 to 12, which further comprises a signal peptide and/or a tag protein;
for example, the fusion protein comprises a signal peptide at its N-terminal;
for example, the signal peptide is a B2M signal peptide, such as a signal peptide set forth in SEQ ID NO: 37;
for example, the fusion protein comprises a tag protein, such as a His tag, at its C-terminal.

14. The fusion protein according to any one of claims 1 to 13, which comprises an amino acid sequence set forth in any one of SEQ ID NOs: 7, 8, 13, 15, 17 and 19;
for example, the fusion protein is encoded by a sequence set forth in any one of SEQ ID NOs: 9, 10, 14, 16, 18, 20.

15. A fusion protein, which comprises a S protein ectodomain sequence of coronavirus, a trimerization domain sequence, and a fluorescent protein.

16. The fusion protein according to claim 15, wherein the fusion protein comprises the S protein ectodomain sequence, the trimerization domain sequence and the fluorescent protein from the N-terminal to the C-terminal.

17. The fusion protein according to claim 15 or 16, wherein the coronavirus is SARS-CoV-2.

18. The fusion protein according to any one of claims 15 to 17, wherein the S protein ectodomain sequence is selected from the amino acid sequences shown below:
(i) a sequence set forth in SEQ ID NO: 21;
(ii) a sequence having a substitution, deletion or addition of one or several amino acids (e.g., a substitution, deletion or addition of 1, 2, 3, 4, 5, 6, 7, 8, 9 or 10 amino acids) as compared to the sequence set forth in SEQ ID NO: 21; or
(iii) a sequence having a sequence identity of at least 80%, at least 85%, at least 90%, at least 91%, at least 92%, at least 93%, at least 94%, at least 95%, at least 96%, at least 97%, at least 98%, or at least 99% as compared to the sequence set forth in SEQ ID NO: 21;
for example, the S protein ectodomain sequence is encoded by a sequence set forth in SEQ ID NO: 22.

19. The fusion protein according to any one of claims 15 to 18, wherein the trimerization domain sequence comprises a sequence set forth in SEQ ID NO: 40;
for example, the trimerization domain sequence is encoded by a sequence set forth in SEQ ID NO:23.

20. The fusion protein according to any one of claims 15 to 19, wherein the trimerization domain sequence and the fluorescent protein are optionally linked by a peptide linker (e.g., a flexible peptide linker);
for example, the peptide linker comprises 1 to 15 contiguous amino acid residues that are identical or different and selected from glycine and serine; for example, the peptide linker is (GₘS)ₙ, wherein m is an integer selected from 1 to 4, and n is an integer selected from 1 to 3; for example, the peptide linker comprises a sequence set forth in SEQ ID NO:6.

21. The fusion protein according to any one of claims 15 to 20, wherein the fluorescent protein is selected from the group consisting of green fluorescent protein, blue fluorescent protein, cyan fluorescent protein, yellow fluorescent protein, orange or red fluorescent protein, near-infrared fluorescent protein, or long Stoke shift fluorescent protein;
for example, the fluorescent protein is a green fluorescent protein, such as mGamillus, mNeonGreen, EGFP, mClover, UnaG, TurboGFP, TagGFP, Venus, EYFP, RFP, iRFP670, mBeRFP, CyOFP1;
for example, the fluorescent protein comprises a sequence set forth in SEQ ID NO: 38 or 39.

22. The fusion protein according to any one of claims 15 to 21, comprising an amino acid sequence selected from the group consisting of:
(1) an amino acid sequence consisting of amino acid residues at the positions 21 to 1502 of the sequence set forth in SEQ ID NO: 26; or
(2) an amino acid sequence consisting of amino acid residues at the positions 21 to 1499 of the sequence set forth in SEQ ID NO: 27.

23. The fusion protein according to any one of claims 15 to 22, which further comprises a signal peptide and/or a tag protein;
for example, the fusion protein comprises a signal peptide at its N-terminal;
for example, the signal peptide is a B2M signal peptide, such as a signal peptide set forth in SEQ ID NO: 37;
for example, the fusion protein comprises a tag protein, such as a His tag, at its C-terminal.

24. The fusion protein according to any one of claims 15 to 23, which comprises an amino acid sequence set forth in SEQ ID NO: 26 or 27;
for example, the fusion protein is encoded by a sequence set forth in SEQ ID NO: 24 or 25.

25. A multimer, which comprises the fusion protein according to any one of claims 15 to 24;
for example, the multimer is a homomultimer;
for example, the multimer is a trimer;
for example, the multimer is a trimer formed from the same fusion protein.

26. An isolated nucleic acid molecule, which comprises a nucleotide sequence encoding the fusion protein according to any one of claims 1 to 24;
for example, the isolated nucleic acid molecule comprises a nucleotide sequence encoding the fusion protein according to any one of claims 1 to 14;
for example, the isolated nucleic acid molecule comprises a nucleotide sequence encoding the fusion protein according to any one of claims 15 to 24.

27. A vector, which comprises the isolated nucleic acid molecule according to claim 26.

28. A host cell, which comprises the isolated nucleic acid molecule according to claim 26 or the vector according to claim 27.

29. A method for preparing the fusion protein according to any one of claims 1 to 14, which comprises, culturing the host cell according to claim 28 under suitable conditions, and recovering the fusion protein from a cell culture; wherein the host cell comprises a nucleotide sequence encoding the fusion protein according to any one of claims 1 to 14.

30. A method for preparing the fusion protein according to any one of claims 15 to 24 or the multimer according to claim 25, which comprises, culturing the host cell according to claim 28 under suitable conditions, and recovering the fusion protein or the multimer from a cell culture; wherein the host cell comprises a nucleotide sequence encoding the fusion protein according to any one of claims 15 to 24;
for example, the fusion protein exists in a multimeric form (e.g., a trimeric form).

31. A kit, which comprises: (i) the fusion protein according to any one of claims 1 to 14; or, (ii) the fusion protein according to any one of claims 15 to 24 or the multimer according to claim 25.

32. The kit according to claim 31, wherein the kit further comprises a cell expressing a recombinant coronavirus receptor, and the recombinant coronavirus receptor comprises a coronavirus receptor and a fluorescent protein fused thereto.

33. The kit according to claim 32, wherein the recombinant coronavirus receptor is selected from the group consisting of ACE2, DPP4, APN and the like.

34. The kit according to claim 32 or 33, wherein the recombinant coronavirus receptor comprises the coronavirus receptor and the fluorescent protein from the N-terminal to the C-terminal.

35. The kit according to any one of claims 32 to 34, wherein the cell stably expresses the recombinant coronavirus receptor.

36. The kit according to any one of claims 32 to 35, wherein, the cell expresses the recombinant coronavirus receptor on its surface.

37. The kit according to any one of claims 32 to 36, wherein the cell comprises a nucleotide sequence encoding the recombinant coronavirus receptor.

38. The kit according to any one of claims 32 to 37, wherein the cell is an adherent cell, such as 293T or H1299 cell.

39. The kit according to any one of claims 32 to 38, wherein the coronavirus receptor is an ACE2 protein, such as a human ACE2 protein;
for example, the human ACE2 protein comprises a sequence set forth in SEQ ID NO: 41;
for example, the human ACE2 protein is encoded by a sequence set forth in SEQ ID NO:30.

40. The kit according to any one of claims 32 to 39, wherein the fluorescent protein contained in the recombinant coronavirus receptor is selected from the group consisting of green fluorescent protein, blue fluorescent protein, cyan fluorescent protein, yellow fluorescent protein, orange fluorescent protein or red fluorescent protein, near-infrared fluorescent protein, or long Stokes shift fluorescent protein;
for example, the fluorescent protein is selected from red fluorescent protein, near-infrared fluorescent protein or long Stokes shift fluorescent protein, such as mRuby3, mApple, FusionRed, mCherry, mScarlet, RFP, iRFP670, mBeRFP or CyOFP1;
for example, the fluorescent protein comprises a sequence set forth in SEQ ID NO:42.

41. The kit according to any one of claims 32 to 40, wherein the recombinant coronavirus receptor comprises a sequence set forth in SEQ ID NO: 32;
for example, the recombinant coronavirus receptor is encoded by a sequence set forth in SEQ ID NO: 31.

42. The kit according to claim 41, wherein the cell comprises a sequence set forth in SEQ ID NO:31.

43. The kit according to any one of claims 32 to 42, which comprises: the fusion protein according to any one of claims 1 to 14, and the cell expressing the recombinant coronavirus receptor.

44. The kit according to claim 43, wherein the fluorescent protein contained in the fusion protein is detectably different from the fluorescent protein in the recombinant coronavirus receptor expressed by the cell.

45. The kit according to claim 43 or 44, wherein the fluorescent protein contained in the fusion protein is a green fluorescent protein (e.g., mGamillus, mNeonGreen, EGFP, mClover, UnaG, TurboGFP, TagGFP, Venus, EYFP, RFP, iRFP670, mBeRFP, CyOFP1); and/or, the fluorescent protein in the recombinant coronavirus receptor expressed by the cell is selected from red fluorescent protein, near-infrared fluorescent protein, or long Stokes shift fluorescent protein (e.g., mRuby3, mApple, FusionRed, mCherry, mScarlet, RFP, iRFP670, mBeRFP or CyOFP1).

46. The kit according to any one of claims 32 to 42, which comprises: the multimer (e.g., trimer) according to claim 25, and the cell expressing the recombinant coronavirus receptor.

47. The kit according to claim 46, wherein the fluorescent protein contained in the monomer forming the multimer is detectably different from the fluorescent protein in the recombinant coronavirus receptor expressed by the cell.

48. The kit according to claim 46 or 47, wherein the fluorescent protein contained in the monomer forming the multimer is a green fluorescent protein (e.g., mGamillus, mNeonGreen, EGFP, mClover, UnaG, TurboGFP, TagGFP, Venus, EYFP, RFP, iRFP670, mBeRFP, CyOFP1); and/or, the fluorescent protein in the recombinant coronavirus receptor expressed by the cell is selected from red fluorescent protein, near-infrared fluorescent protein, or long Stokes shift fluorescent protein (e.g., mRuby3, mApple, FusionRed, mCherry, mScarlet, RFP, iRFP670, mBeRFP, or CyOFP1).

49. The kit according to any one of claims 31 to 48, which further comprises a solid support;
for example, the solid support is selected from a microtiter plate (e.g., a microwell plate or an ELISA plate);
for example, the solid support is suitable for fluorescence measurement;
for example, the cell is immobilized on a surface of the solid support.

50. A method for evaluating a fusion inhibitory activity of a fusion inhibitor of a coronavirus, and/or for screening a fusion inhibitor of coronavirus, which comprises using the fusion protein according to any one of claim 1 to 24, the multimer according to claim 25, or the kit according to any one of claims 31 to 49.

51. The method according to claim 50, which comprises:
(1) in the presence of a reagent to be tested, contacting a detection reagent with the cell expressing a recombinant coronavirus receptor as defined in any one of claims 32 to 48, wherein the detection reagent is selected from: the fusion protein according to any one of claims 1 to 14 or the multimer according to claim 25; wherein the fluorescent protein contained in the detection reagent is detectably different from the fluorescent protein in the recombinant coronavirus receptor expressed by the cell;
(2) measuring a fluorescence intensity of a cytoplasmic region of the cell, wherein the fluorescence intensity is a fluorescence intensity of the fluorescent protein contained in the detection reagent.

52. The method according to claim 51, wherein the method further comprises:
(3) comparing the measured value in step (2) with a fluorescence intensity measured in the absence of the reagent to be tested, and obtaining the following ratio: (measured value in the absence of the reagent to be tested - measured value of step (2)) / measured value in the absence of the reagent to be tested.

53. The method according to claim 52, wherein the method further comprises:
(4) generating a dose-response curve of the reagent to be tested on the basis of the ratio obtained in step (3), and thereby obtaining EC₅₀; evaluating a fusion inhibitory activity against the coronavirus of the reagent to be tested according to the EC₅₀.

54. The method according to any one of claims 51 to 53, wherein the cell described in step (1) is immobilized on a surface of a solid support;
for example, the solid support is selected from a microtiter plate (e.g., a microwell plate or an ELISA plate);
for example, the solid support is suitable for fluorescence measurements.

55. The method according to claim 53, wherein the EC₅₀ of step (4) is obtained by the following method: repeating steps (1) to (3) with a series of samples comprising different amounts of the reagent to be tested, thereby producing a dose-response curve of the reagent to be tested and thereby determining EC₅₀.

56. The method according to any one of claims 51 to 55, wherein the number of the cell in step (1) and step (2) is plural;
for example, the number of the cell determined in step (2) is not less than 100, for example, not less than 500, not less than 800, or not less than 1000.

57. The method according to claim 56, wherein the fluorescence intensity measured in step (2) is an average fluorescence intensity.

58. The method according to any one of claims 51 to 57, wherein step (2) may further comprise: measuring a total fluorescence intensity of the cell wherein the fluorescence intensity is a fluorescence intensity of the fluorescent protein contained in the fusion protein or multimer; and comparing the fluorescence intensity of the cytoplasmic region with the total fluorescence intensity of the cell to which it belongs, and obtaining the ratio of the two, wherein the ratio can be used as a ratio reflecting cell uptake of probe, and as a measured value obtained in step (2) for subsequent analysis.

59. The method according to any one of claims 51 to 58, wherein step (2) further comprises: measuring a fluorescence intensity of the cell membrane region of the cell wherein the fluorescence intensity is a fluorescence intensity of the fluorescent protein contained in the recombinant coronavirus receptor expressed by the cell.

60. The method according to claim 59, wherein step (2) further comprises: comparing the fluorescence intensity of cell membrane region between different experiment batches, or between different test wells of the same experiment batch, wherein the fluorescence intensity is a fluorescence intensity of the fluorescent protein contained in the recombinant coronavirus receptor expressed by the cell, and the value can be used for correction of variation between different batches or different test wells.

61. The method according to claim 59 or 60, wherein step (2) further comprises: comparing the fluorescence intensity of cytoplasmic region with the fluorescence intensity of cell membrane region, and obtaining a ratio between the two, and the ratio being used as a calibrated value of the measured value for a subsequent step; wherein, the fluorescence intensity of cytoplasmic region is a fluorescence intensity of the fluorescent protein contained in the fusion protein or the multimer, and the fluorescence intensity of cell membrane region is a fluorescence intensity of the fluorescent protein contained in the recombinant coronavirus receptor.

62. The method according to any one of claims 51 to 61, wherein the measuring in step (2) is performed by a fluorescence microscope or a high content imaging system.

63. The method according to any of claims 51 to 62, wherein no washing step is comprised between step (1) and step (2).

64. The method according to any one of claims 51 to 63, wherein the coronavirus uses ACE2 (e.g., human ACE2) as a cell receptor.

65. The method according to claim 64, wherein the coronavirus is selected from SARS-CoV-2 and/or SARS-CoV-1; for example, the coronavirus is SARS-CoV-2.

66. The method according to any one of claims 51 to 65, wherein the fusion inhibitor against coronavirus is selected from a reagent capable of blocking or inhibiting the binding between a coronavirus S protein RBD and a cell receptor (e.g., human ACE2) thereof.

67. The method according to claim 66, wherein the fusion inhibitor against coronavirus is selected from a reagent capable of specifically binding to a coronavirus S protein RBD or specifically binding to a coronavirus cell receptor (e.g., human ACE2).

68. The method according to claim 66 or 67, wherein the reagent is selected from a neutralizing antibody or blocking antibody that specifically binds to the coronavirus S protein RBD, a polypeptide or protein derived from the coronavirus cell receptor (e.g., ACE2) (e.g., a polypeptide or protein comprising an extracellular domain of ACE2), or a polypeptide or protein derived from a RBD protein or S1 protein of a coronavirus (e.g., a polypeptide or protein comprising the full-length sequence of the RBD protein or the S1 protein or an active fragment thereof).

69. Use of the fusion protein according to any one of claims 1 to 24, the multimer according to claim 25, or the kit according to any one of claims 31 to 49, in the manufacture of a detection reagent for evaluating an activity of a fusion inhibitor against a coronavirus, and/or for screening a fusion inhibitor against a coronavirus.

70. The use according to claim 69, wherein the detection reagent evaluates the activity of the fusion inhibitor against the coronavirus and/or screens the fusion inhibitor against the coronavirus by the method according to any one of claims 51 to 68.

71. The use according to claim 69 or 70, wherein the detection reagent is further used for screening a drug capable of preventing and/or treating a coronavirus infection or a disease related to a coronavirus infection.

72. The use according to any one of claims 69 to 71, wherein the coronavirus uses ACE2 (e.g., human ACE2) as a cell receptor.

73. The use according to claim 72, wherein the coronavirus is selected from SARS-CoV-2 and/or SARS-CoV-1; for example, the coronavirus is SARS-CoV-2.
